(19) Europäisches Patentamt
European Patent Office
Office européen des brevets

(11) **EP 3 018 213 A1**

(12) **EUROPEAN PATENT APPLICATION**

(43) Date of publication:
**11.05.2016 Bulletin 2016/19**

(51) Int Cl.:
**C12Q 1/68** (2006.01)

(21) Application number: **14191764.1**

(22) Date of filing: **04.11.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Genesupport SA**
**1700 Fribourg (CH)**

(72) Inventor: **Conrad, Bernard**
**1700 Fribourg (CH)**

(74) Representative: **Rentsch Partner AG**
**Rechtsanwälte und Patentanwälte**
**Fraumünsterstrasse 9**
**Postfach 2441**
**8022 Zürich (CH)**

(54) **Method for determining the presence of a biological condition by determining total and relative amounts of two different nucleic acids**

(57)    The present invention relates to a method for determining the presence of a biological condition associated with a nucleic acid species N1 in a test biological sample obtained from a patient, said biological sample comprising two nucleic acid species N1 and N2, wherein the method comprises:

a) determining the total amount [N1 + N2] and comparing said total amount to at least one discrimination threshold T;

b) determining the relative amount N1:N2 and comparing said relative amount to at least one discrimination threshold R specific for the method of step (a); and

c) on the basis of the comparisons carried out in step (a) and (b), determining the presence of the biological condition.

The method generally comprises extraction of the total nucleic acids from a sample; optionally enrichment of the species N1, possibly by size selection; attribution of sequenced nucleic acids larger than 50 bp to individual chromosomes; and comparison of sequence reads with references.

Figure 1

**Description**

The present invention relates to the field of biomedical analysis, and specifically describes methods for determining the presence of a biological condition through the analysis of nucleic acids in a biological sample. In particular, the present invention describes methods for determining the presence of a biological condition in a biological sample comprising two nucleic acid species, wherein the biological condition is associated with only one of the nucleic acid species. More specifically, the present invention relates to methods in which a biological condition associated with one of two nucleic acid species, is detected by quantitatively assessing the sum of the amounts of the two nucleic acid species and determining whether this sum is above a predefined threshold, and by determining whether the relative amounts of the two species is above another predefined threshold, specific for the quantitative assessment method.

Background

Since the discovery of fetal DNA fragments in the plasma of pregnant women (Lo et al., 1997), much progress has been made in the use of cell-free fetal DNA or RNA for predictive and diagnosis purposes (Bianchi, 2012; Hui and Bianchi, 2013).

Cell-free fetal DNA is for example used to diagnose fetal aneuploidies, such as the main trisomies 13, 18 and 21. The diagnosis generally relies on a common estimation of fetal and maternal sequences originating from a particular chromosome. A fetal aneuploidy, for example a trisomy 21, will result in a small, but detectable excess of sequences from the chromosome 21. A key parameter in such methods is the fetal fraction, i.e. the proportion of cell-free DNA originating from the fetus within the biological sample drawn from the mother. This parameter has a tremendous importance in the accuracy of non-invasive prenatal testing (NIPT) methods, such as those based on the detection of an excess chromosomal dosage. In most cases, the chromosomal dosage resulting from a fetal aneuploidy is directly related to the fraction of fetal cell-free DNA. For example a sample containing 4% DNA from a fetus affected by trisomy 21 should exhibit a 2% increase in the proportion of reads from chromosome 21, as compared to a normal fetus.

The contribution of cell-free fetal DNA in maternal plasma has been shown to be of 11.4%, on average, with an interquartile range of 8.5-14.6%, (Ashoor et al., 2012). This average value however hides considerable variations between pregnancies, as well as in the course of a single pregnancy. With a view to determining the cause of these variations and a way to predict the fetal contribution to cell-free DNA, fetal fraction has been correlated with various biological parameters in the literature. A repeated finding is that fetal fraction decreases as maternal weight increases. It has been shown, for instance, that the proportion of pregnancies with fetal fraction below 4% increases with maternal weight from 0.7% at 60 kg to 7.1% at 100 kg and 51.1% at 160 kg (Ashoor et al., 2013). Another important finding is that fetal fraction increases with the gestational stage (Wang et al., 2013). Fetal fraction has also been correlated with certain placental markers (Ashoor et al., 2012, 2013). The fetal fraction furthermore differs among aneuploidies. Compared to euploid fetuses the fetal fraction is slightly higher for trisomy 21, but lower for trisomy 18 and monosomy X, and is lowest for trisomy 13 (Dar et al., 2014).

Little literature has been published on the use of reliable fetal fraction thresholds, e.g. thresholds above which the diagnosis of a fetal condition would be considered as meaningful, and below which this diagnosis should be disregarded. In one of the rare articles to address this issue, it is estimated that at a throughput of about 10 million useful reads, detection of fetal trisomy 21 is enabled with a level of confidence $\alpha<0.001$ (i.e. a statistical chance to yield a false result of 1/1'000) when the fetal fraction is above 3.9% fetal DNA (Fan and Quake, 2010). This calculation was made in a particular diagnosis scheme, using a specific protocol, including the removal of the GC-bias. Moreover, and most importantly, it was based on several hypotheses, including the assumption that the sampling of sequences follows the Poisson distribution. Altogether, the value given by Fan and Quake is based on theory, and not necessarily adapted to the diagnosis method effectively used in every laboratory. In spite of its highly theoretical nature, the value of 4% (approximation derived from the 3.9% calculated in Fan and Quake, 2010) has rapidly become a standard in the field. As evident from the literature (Ashoor et al., 2013; Sparks et al., 2012; Srinivasan et al., 2013; Struble et al., 2014; Wang et al., 2013), the person skilled in the field now considers that a fetal fraction above 4% is synonymous with a meaningful result, whilst a fetal fraction below 4% corresponds to a "no-call" sample, for which no meaningful diagnosis can be established. This use of the threshold of 4% nevertheless does not necessarily correspond to the reality of the diagnosis method used in the laboratory.

For this reason, there is a need, in the field of NIPT, for a robust and reliable way to assess the causal implication of fetal fraction on the reliability of the diagnosis. This need exists in other fields concerned with the detection of a biological condition through the quantitative analysis of nucleic acids, such as the diagnosis of cancer, and thus concerns any method based on the common detection of two nucleic acid species to determine the presence of a biological condition associated with only one of the nucleic acids species. In this more general case, it may be desirable to assess the causal implication of the relative amount of the two nucleic acid species, on the reliability of the detection method.

The present invention responds to this demand, and provides a method for determining the presence of a biological condition associated with a nucleic acid species N1 in a test biological sample obtained from a patient, said test biological sample comprising two nucleic acid species N1 and N2, wherein the method comprises:

> a) determining the total amount [N1 + N2] and comparing said total amount to at least one discrimination threshold T;
> b) determining the relative amount N1:N2 and comparing said relative amount to at least one discrimination threshold R specific for the method of step (a); and
> c) on the basis of the comparisons carried out in step (a) and (b), determining the presence of the biological condition.

In one embodiment, the discrimination threshold R is defined by using one or more reference samples. Specifically, defining the discrimination threshold R can involve the determination of the relative amount N1:N2 of one or more reference samples. In a specific embodiment, the discrimination threshold R is directly derived from, in particular is, the relative amount N1:N2 of a reference sample, or the average relative amount N1:N2 of more than one reference samples. Hence, in one embodiment, the comparison in step (b) involves comparing the relative amount N1:N2 of the test biological sample to the relative amount N1:N2 of one or more reference samples.

Accordingly, in one embodiment, step (b) consists in or comprises determining the relative amount N1:N2 and comparing the relative amount N1:N2 of the test biological sample to the relative amount N1:N2 of one or more reference samples.

In one embodiment, the reference samples are selected by comparing their total amount [N1 + N2] with respect to the discrimination threshold T. In particular, the reference samples can be selected on the basis of the distance of their total amount [N1 + N2] to the discrimination threshold T.

In one embodiment, the nucleic acid species N1 and N2 are cell free nucleic acids.

In one embodiment, the nucleic acid species N1 are fetal nucleic acids and the nucleic acid species N2 are maternal nucleic acids. In another embodiment, the nucleic acids N1 are derived from a tumor cell and/or tissue, and the nucleic acid species N2 are derived from a non-tumor cell and/or tissue.

In one embodiment, if the relative amount N1:N2 is below the discrimination threshold R:

- the relative amount N1:N2 is enriched; and/or

- the biological condition is detected on another biological sample drawn from the same patient.

In one embodiment, the relative amount N1:N2 is enriched, for example by size selection of the nucleic acids in the test biological sample. The size selection can comprise enriching the nucleic acids with a maximum length of 200 nucleotides or less, or 175 nucleotides or less, or 150 nucleotides or less. The size selection can be carried out by electrophoresis, preferably on a gel.

In one embodiment, the relative amount N1:N2 is enriched by at least 1.5 fold or at least 3 fold or at least 5 fold.

In one embodiment, the relative amount N1:N2 is determined by analyzing one or more nucleic acid sequences specific to N1 and/or by bioinformatics analysis of data generated by sequencing, preferably massively parallel sequencing.

In one embodiment, the relative amount N1:N2 is deduced from a nucleic acid excess or lack originating from a chromosomal or subchromosomal copy number variation.

In one embodiment, the test biological sample is obtained from an overweight patient, i.e. a patient with a body mass index (BMI) of 25 or more. In one embodiment, the test biological sample is obtained from an obese patient, i.e. a patient with a body mass index (BMI) of 30 or more.

In one embodiment, determining the total amount [N1 + N2] comprises:

- sequencing the nucleic acids species N1 and N2 from the test biological sample;

- aligning the sequences to a reference genome or a portion thereof; and

- from the aligned sequences, determining the total amount [N1 + N2].

In one embodiment, the method according to the invention comprises, prior to the massively parallel sequencing:

- extracting the nucleic acids species N1 and N2 from the test biological sample; and

- preparing a sequencing library from the extracted nucleic acids.

In one embodiment, the discrimination threshold T is defined by using one or more reference samples. Specifically, defining the discrimination threshold T can involve the determination of the total amount [N1 + N2] of one or more reference samples. In a specific embodiment, the discrimination threshold T is directly derived from, in particular is, the total amount [N1 + N2] of a reference sample, or the average total amount [N1 + N2] of more than one reference samples. Hence, in one embodiment, the comparison in step (a) involves comparing the total amount [N1 + N2] of the test biological sample to the total amount [N1 + N2] of one or more reference samples.

Accordingly, in one embodiment, step (a) consists in or comprises determining the total amount [N1 + N2] and comparing the total amount [N1 + N2] of the test biological sample to the total amount [N1 + N2] of one or more reference samples.

In one embodiment, the biological condition is cancer. In another embodiment, the biological condition is a fetal chromosomal or subchromosomal copy number variation, preferably trisomy 13, 18 or 21. Where the biological condition is a fetal chromosomal copy number variation, in particular trisomy 13, 18 or 21 or monosomy X, the comparison in step (b) can involve comparing the relative amount N1:N2 of the test biological sample to the relative amount N1:N2 of one or more reference samples obtained from a trisomy 13 pregnancy.

Another aspect of the invention is a method for selecting a reference sample for the relative amount N1:N2, wherein the method for selecting a reference sample comprises:

- providing a set of biological samples;

- selecting a reference sample for the relative amount N1:N2 by comparing its total amount [N1 + N2] with the discrimination threshold T.

In one embodiment, the discrimination threshold T is itself defined by using one or more reference samples for the total amount [N1 + N2].

Hence, an embodiment of the invention is a method for selecting a reference sample for the relative amount N1:N2, wherein the method for selecting a reference sample comprises:

- providing a set of biological samples;

- selecting a reference sample for the relative amount N1:N2 by comparing its total amount [N1 + N2] with the total amount [N1 + N2] of one or more reference samples for the total amount [N1 + N2].

Another aspect of the invention is a method for defining a discrimination threshold R for a relative amount N1:N2 of two nucleic acid species N1 and N2 in a test biological sample, said discrimination threshold R being specific for a method for determining the presence of a biological condition associated with N1, said method for determining the presence of a biological condition comprising the comparison of the total amount [N1 + N2] in the test biological sample to at least one discrimination threshold T, wherein said method for defining a discrimination threshold R comprises providing at least one reference sample, and defining the discrimination threshold R from the relative amount N1:N2 in the reference sample.

In one embodiment, the reference sample is selected by comparing its total amount [N1 + N2] with the discrimination threshold T.

In one embodiment, the reference sample is obtained from a trisomy 13 pregnancy.

In one embodiment, the relative amount N1:N2 is determined by analyzing one or more nucleic acid sequences specific to N1 and/or by bioinformatics analysis of data generated by sequencing, in particular massively parallel sequencing.

In one embodiment, the relative amount N1:N2 is deduced from a nucleic acid excess or lack originating from a chromosomal or subchromosomal copy number variation.

Another aspect of the invention is a method for assessing the reliability of a method for determining the presence of a biological condition associated with a nucleic acid species N1 in a test biological sample comprising two nucleic acid species N1 and N2, said method for determining the presence of a biological condition comprising the comparison of the total amount of [N1 + N2] to at least one discrimination threshold T, wherein the reliability of the method for determining the presence of a biological condition is assessed by comparing the relative amount N1:N2 in the test biological sample to at least one discrimination threshold R specific for the method for determining the presence of a biological condition.

Another aspect of the invention is a method for assessing the reliability of a method for determining the presence of a biological condition associated with a nucleic acid species N1 in a test biological sample comprising two nucleic acid species N1 and N2, said method for determining the presence of a biological condition comprising the comparison of the total amount of [N1 +N2] to a discrimination threshold T, wherein the method for assessing the reliability of the method for determining the presence of a biological condition comprises:

- providing a set of biological samples obtained from a set of patients;

- selecting at least one reference sample from the set of biological samples, on the basis of a comparison of its total amount [N1 + N2] with the discrimination threshold T.

- assessing the reliability of the method for determining the presence of a biological condition by comparing the relative amount N1:N2 in the test biological sample to the relative amount N1:N2 in the reference sample.

Another aspect of the invention is a method for determining the presence of a biological condition associated with a nucleic acid species N1 in a test biological sample obtained from a patient, comprising two nucleic acid species N1 and N2, said method comprising:

- providing a set of biological samples obtained from a set of patients;

- selecting at least one reference sample from the set of biological samples by comparing its total amount [N1 + N2] with at least one discrimination threshold T;

- determining the total amount [N1+N2] in the test biological sample and comparing said total amount to the discrimination threshold T;

- determining the relative amount N1:N2 in the test biological sample and comparing said relative amount to the relative amount N1:N2 in the reference sample(s); and

- on the basis of the results of both comparisons, determining the presence of the biological condition.

In a specific embodiment, the present invention relates to a method for determining the presence of a fetal aneuploidy, in particular trisomy 13, 18 or 21, from a test biological sample obtained from a pregnant woman, comprising cell-free fetal nucleic acids and cell-free maternal nucleic acids, said method comprising:

a) determining the total amount of cell-free fetal and maternal nucleic acids originating from a chromosome or chromosomal region and comparing said total amount to at least one discrimination threshold T;

b) determining the relative amount of cell-free fetal nucleic acids to cell-free maternal nucleic acids and comparing said relative amount to at least one discrimination threshold R specific for the method of step (a); and

c) on the basis of the comparisons carried out in step (a) and (b), determining the presence of a fetal aneuploidy.

In a specific embodiment, the present invention relates to a method for determining the presence of a cancer from a test biological sample obtained from a subject, comprising cell-free tumor-derived nucleic acids and cell-free non tumor-derived nucleic acids, said method comprising:

a) determining the total amount of cell-free tumor-derived and non tumor-derived nucleic acids originating from a chromosome or chromosomal region and comparing said total amount to at least one discrimination threshold T;

b) determining the relative amount of cell-free tumor-derived nucleic acids to cell-free non tumor-derived nucleic acids and comparing said relative amount to at least one discrimination threshold R specific for the method of step (a); and

c) on the basis of the comparisons carried out in step (a) and (b), determining the presence of a cancer.

In one embodiment, determining an amount (relative or total) of nucleic acids means determining an amount of sequences of said nucleic acids. In a specific embodiment, the nucleic acids are DNA.

The present invention also relates to a computer program product for implementing one or more steps of:

- the method for determining the presence a biological condition according to the invention; and/or

- the method for selecting a reference sample useful to define a discrimination threshold R for a relative amount N1 :N2 of two nucleic acid species N1 and N2 in a biological sample, according to the invention; and/or

- the method for defining a discrimination threshold R for a relative amount N1:N2 of two nucleic acid species N1 and N2 in a biological sample, according to the invention; and/or

- the method for assessing the reliability of a method for determining the presence of a biological condition associated with a nucleic acid species N1 in a biological sample comprising two nucleic acid species N1 and N2, according to the invention.

The present invention also relates to a kit comprising one or more of:

- one or more compositions and/or a kit for extracting cell-free DNA;

- a set of reference samples useful to define a discrimination threshold R for a relative amount N1:N2 of two nucleic acid species N1 and N2 in a biological sample, according to the invention;

- a set of discrimination thresholds T obtainable according to the method according to the present invention, optionally included in a physical support, such as a computer readable media;

- a set of discrimination thresholds R obtainable according to the method according to the present invention, optionally included in a physical support, such as a computer readable media;

- a computer program product according to the invention.

According to a preferred embodiment, the kit according to the invention:

- a set of reference samples useful to define a discrimination threshold R for a relative amount N1:N2 of two nucleic acid species N1 and N2 in a biological sample, according to the invention;

- and / or a set of discrimination thresholds R obtainable according to the method according to the present invention, optionally included in a physical support, such as a computer readable media.

Brief Description of the Drawings

**Figure 1:** Diagram showing an exemplary workflow of a method for determining the presence of a biological condition according to the invention.

**Figure 2:** Diagram showing an exemplary workflow of a method for determining the presence of a biological condition according to the invention, in the specific context of aneuploidy diagnosis. The steps are grouped into 3 modules M1 (DNA extraction), M2 (library construction and sequencing) and M3 (bioinformatics).

**Figure 3:** Size distribution of DNA fragments in a plasma sample obtained from a pregnant woman. Lighter line: fetal DNA molecules (paired-end sequences). Darker line: total (maternal + fetal) DNA (paired-end sequences).

**Figure 4:** Correlation between the fetal fraction and the signal intensity for trisomy 21. The signal intensity corresponds to the count of sequences (UEMs) matched to chromosome 21.

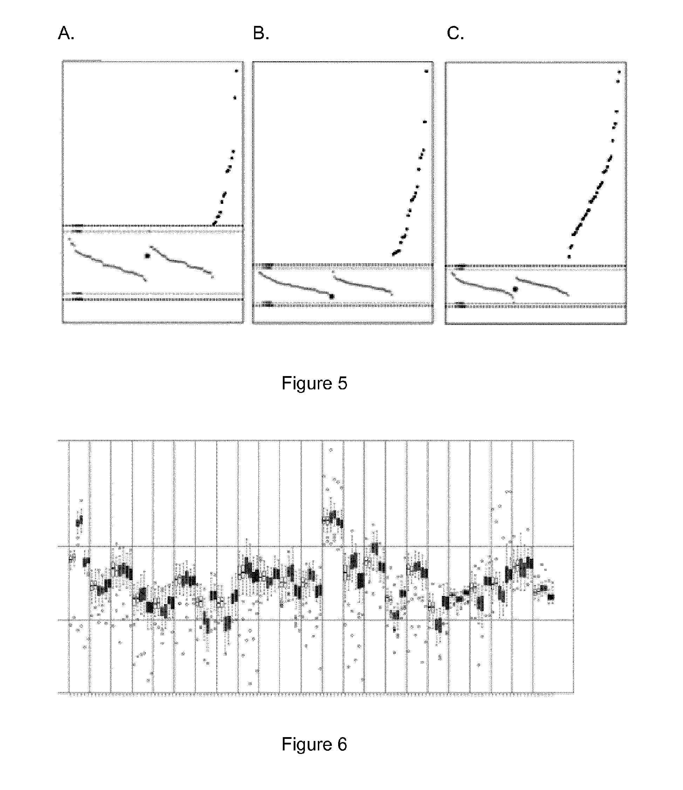

**Figure 5:** Exemplary tests for trisomy 13 (A), trisomy 18 (B) and trisomy 21 (C). Plot A is magnified in comparison with plots B and C. The plots represent the count of sequences (UEMs) matched, respectively to chromosome 13 (Figure 5A), 18 (Figure 5B) and 21 (Figure 5C), for a test sample, and for different sets of reference samples. The two dotted lines correspond to probability thresholds T of 1/10'000 (bolder dotted line), respectively 1/1'000 (lighter dotted line), i.e. only one sample over 10'000, respectively 1'000, statistically exceeds this threshold. The test sample corresponds to the bolder spot, in the middle of each plot. The spots between the dotted lines, on the left and on the right of the test sample, correspond to euploid reference samples. The spots above the dotted lines, on the right of each plot, correspond to aneuploid reference samples (respectively trisomy 13, 18 and 21 samples). A comparison of the aneuploid reference samples of each test shows that the lowest signal intensity within the aneuploid reference samples is lower for trisomy 13 than for trisomy 18 and 21.

**Figure 6:** Verification of the robustness of the diagnosis of trisomy 13, for sample GWX-311 selected as a reference sample defining a discrimination threshold R. The vertical lanes represent chromosome 1-22 and X. In each lane, 6 values are plotted, which represent a comparison between the count of sequences (UEMs) for a chromosome of sample GWX-311 with respect to different sets of reference samples. For each chromosome, 6 comparisons with 6 different sets of reference samples are carried out, resulting in 6 values plotted in each lane. Moreover, each plotted value is obtained from 47 independent determinations and is represented in the form of a box-and-whisker plot, showing the maximum and minimal values as well as the interquartile range. A chromosome is found deviant from normality when its UEM count exceeds the indicated threshold T for all 6 comparisons. The threshold T (dotted line) corresponds to a probability of 1/1'000, i.e. only one sample over 1000 statistically exceeds this threshold.

Sample GWX-311 is affected by a trisomy 13, as revealed by the fact that it exceeds the threshold of 1/1'000 for all 6 comparisons carried out for chromosome 13. The distance to the threshold is however minimal when compared to other trisomic samples. The minimal distance to the threshold T indicates that this sample can be selected as a reference sample for the relative amount N1:N2, thereby defining a fetal fraction discrimination threshold R. This reference sample can be used for comparison of the fetal fraction of other samples to be tested.

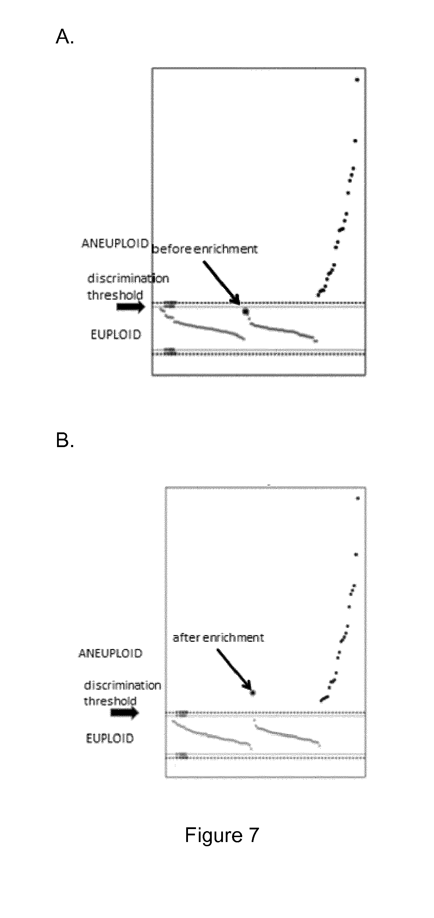

**Figure 7:** result of a test for chromosomal aneuploidy, on chromosome 18 of sample 'GWX-2429', before (A) and after (B) enrichment of the fetal fraction of a sample having a fetal fraction below the threshold value R. The plots represent the count of sequences (UEMs) matched, respectively to chromosome 18, for the test sample, and for a set of reference samples. The two dotted lines correspond to probability thresholds T of 1/10'000 (bolder dotted line), respectively 1/1'000 (lighter dotted line), i.e. only one sample over 10'000, respectively 1'000, statistically exceeds this threshold. The black bold spot, in the middle of each plot, represents the test sample. The other spots represent euploid reference samples (samples between the dotted lines) and aneuploid reference samples (samples on the right, above the dotted lines). The discrimination thresholds T of 1/1'000 and 1/10'000 are shown as dotted lines (light dots: 1/1'000; bold dots: 1/10'000).

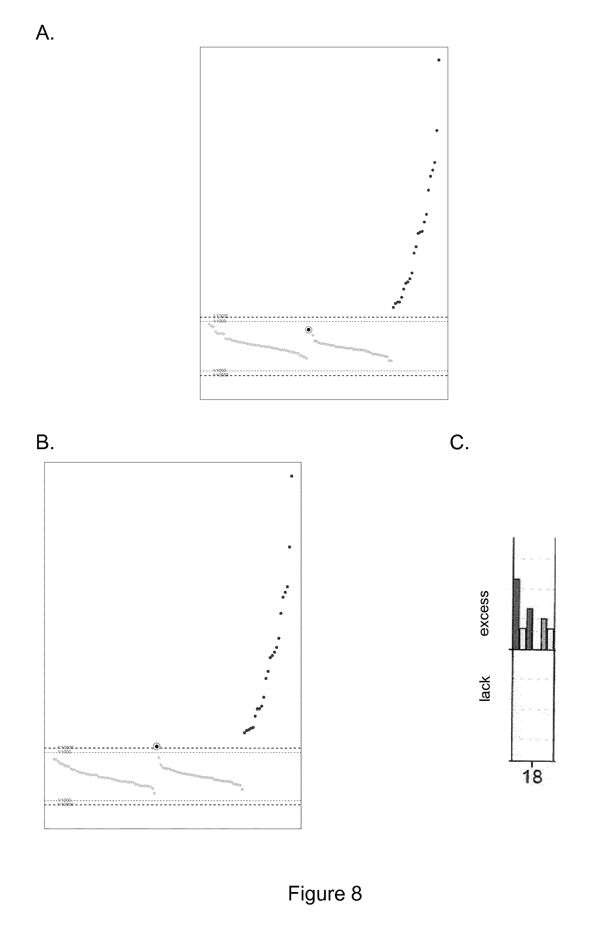

**Figure 8:** result of a test for chromosomal aneuploidy, on chromosome 18 of sample 'GWX-2488', before (A) and after (B) and (C) enrichment of the fetal fraction.

8A: single comparison with one reference set, before enrichment of the fetal fraction. The black bold spot, in the middle of each plot, represents the test sample. The other spots represent euploid reference samples (samples between the dotted lines) and aneuploid reference samples (samples on the right, above the dotted lines). The discrimination thresholds T of 1/1'000 and 1/10'000 are shown as dotted lines (light dots: 1/1'000; bold dots: 1/10'000). Before enrichment of the fetal fraction, the sample '2488' appears to be euploid for chromosome 18, the signal for chromosome 18 being under the 1/1'000 threshold.

8B: single comparison with one reference set (same as Figure 8A), after enrichment of the fetal fraction. The signal for chromosome 18 is just above the 1/1'000 threshold.

8C: 6 comparisons with 6 reference sets, for chromosome 18, after enrichment of the fetal fraction (one of the plotted values correspond to the comparison shown in Figure 8B). The dotted lines represent confidence intervals (discrimination thresholds T) of 1/1'000, 1/1'000'000 and 1/1'000'000'000 with respect to the euploid samples of the reference sets. A chromosome is considered aneuploid if it exceeds the 1/1'000 discrimination threshold for all 6 comparisons with the 6 reference sets. The enrichment of the fetal fraction does not reveal a trisomy 18, as evidenced by the fact that only 3 bars out of 6 exceed the discrimination threshold T of 1/1'000.

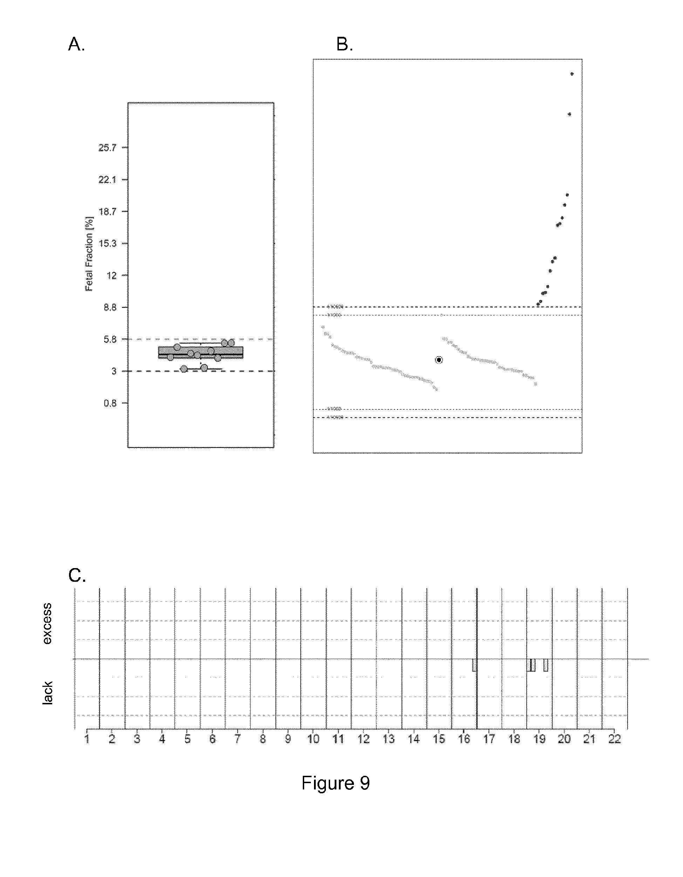

**Figure 9:** result of a test for chromosomal aneuploidy for a negative sample (sample DPA-3932), including a determination of the fetal fraction.

9A: determination of the fetal fraction. 10 individual determinations are shown as individual spots, and as a whisker-and-box plot, showing the interquartile range. The lower dotted line represents the fetal fraction threshold R of 3%, which is equivalent to the lowest-validated chromosomal dosage in a given reference set. Samples with a fetal fraction above the threshold R are considered as having a sufficient fetal fraction for a reliable diagnosis. The upper dotted line represents the fetal fraction of 5.8%. This upper threshold includes about 10% of the lowest fetal fractions. The estimated fetal fraction for the tested sample is $4.4 \pm 0.8$ %.

9B: representation of the count of sequences (UEMs) matched to chromosome 13. The black bold spot represents the test sample. The other spots represent euploid reference samples (samples between the dotted lines) and aneuploidy reference samples (samples above the dotted lines, on the right) reference samples. The discrimination thresholds of 1/1'000 and 1/10'000 are shown as dotted lines (light dotted line: 1/1'000; bolder dotted line: 1/10'000).

9C: representation of the count of sequences (UEMs) for all autosomes. The vertical lanes represent chromosome 1-22. In each lane, 6 values are plotted, which represent a comparison between the count of sequences (UEMs) for a chromosome of the tested sample with respect to different sets of reference samples. For each chromosome, 6 comparisons with 6 different sets of reference samples are carried out, resulting in 6 values plotted in each lane. The dotted lines represent confidence intervals (discrimination thresholds) of 1/1'000, 1/1'000'000 and 1/1'000'000'000 with respect to the euploid samples of the reference sets. A chromosome is considered aneuploid if it exceeds the 1/1'000 discrimination threshold for all 6 comparisons with the 6 reference sets. In the present case, no significant deviation from euploidy is revealed.

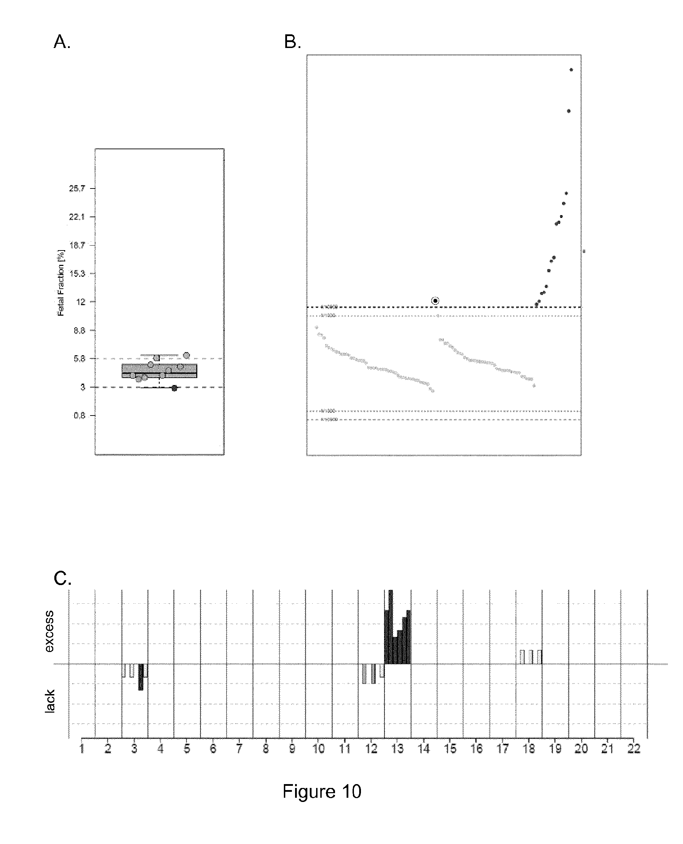

**Figure 10:** result of a test for chromosomal aneuploidy for a sample positive for trisomy 13 (sample GWX-3522), including a determination of the fetal fraction.

10A: determination of the fetal fraction. 10 individual determinations are shown as individual spots, and as a whisker-and-box plot, showing the interquartile range. The lower dotted line represents the fetal fraction threshold R of about 3%, which is equivalent to the lowest-validated chromosomal dosage in a given reference set. Samples with a fetal fraction above the threshold R are considered as having a sufficient fetal fraction for a reliable diagnosis. The upper dotted line represents the fetal fraction of 5.8%. This upper threshold includes about 10% of the lowest fetal fractions. The estimated fetal fraction for the tested sample is $4.5 \pm 1$%.

10B: representation of the count of sequences (UEMs) matched to chromosome 13. The black bold spot represents the test sample. The other spots represent euploid reference samples (samples between the dotted lines) and aneuploid reference samples (samples above the dotted lines, on the right). The discrimination thresholds T of 1/1'000 and 1/10'000 are shown as dotted lines (light dotted line: 1/1'000; bolder dotted line: 1/10'000).

10C: representation of the count of sequences (UEMs) for all autosomes (representation similar to Fig. 9C). The lane "chromosome 13" reveals a trisomy 13, as evidenced by a significant excess (>1/1'000) for all 6 comparisons with 6 reference sets of samples.

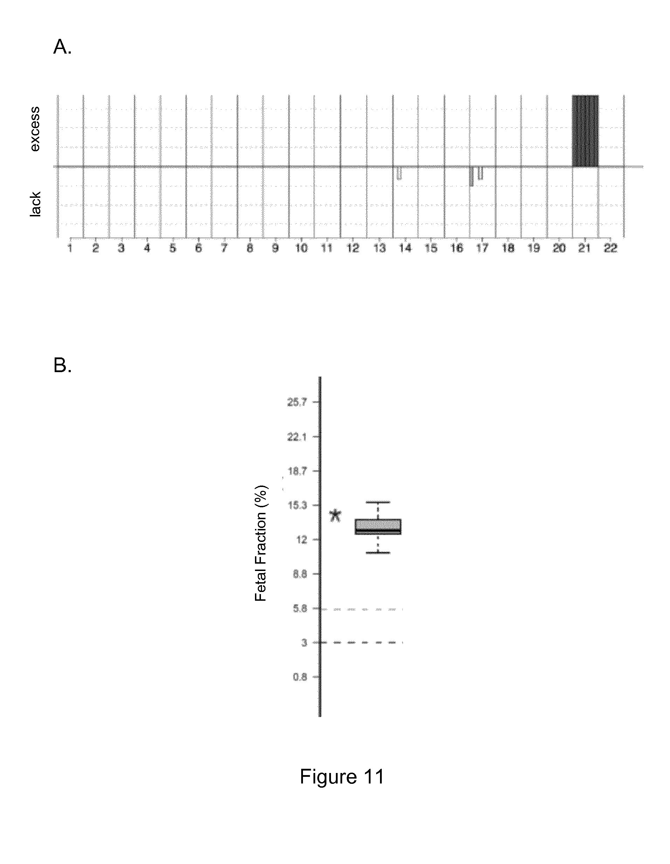

**Figure 11:** example of correlation between the aneuploidy signal intensity and the fetal fraction (sample TPV-113).

11A: result of a test for chromosomal aneuploidy, for a sample positive for chromosome 21, with a high signal intensity (representation similar to Fig. 9C). The intensity for chromosome 21 exceeds the 1/1'000'000'000 threshold (upper dotted line) for all 6 reference sets.

11B: determination of the fetal fraction (mean of 10 determinations). The lower dotted line represents the fetal fraction threshold R of about 3%, which is equivalent to the lowest-validated chromosomal dosage in a given reference set. Samples with a fetal fraction above the threshold R are considered as having a sufficient fetal fraction for a reliable diagnosis. The upper dotted line represents the fetal fraction of 5.8%. This threshold includes about 10% of the lowest fetal fractions.

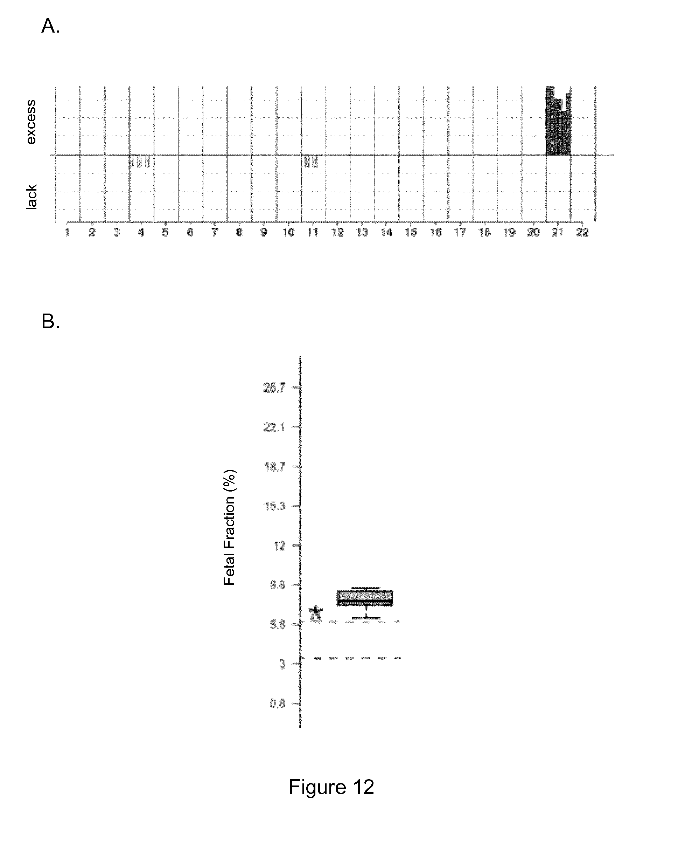

**Figure 12:** example of a correlation between the aneuploidy signal intensity and the fetal fraction (sample TPV-116).

12A: result of a test for chromosomal aneuploidy, for a sample positive for chromosome 21, with a lower signal intensity than the sample of Figure 11 (representation similar to Fig. 9C). The intensity for chromosome 21 exceeds the 1/1'000'000'000 threshold (upper dotted line) for only 3 reference sets.

9B: determination of the fetal fraction (mean of 10 determinations. The lower dotted line represents the fetal fraction threshold R of 3%, which is equivalent to the lowest-validated chromosomal dosage in a given reference set. Samples with a fetal fraction above the threshold R are considered as having a sufficient fetal fraction for a reliable diagnosis. The upper dotted line represents the fetal fraction of 5.8%. This threshold includes about 10% of the lowest fetal fractions.

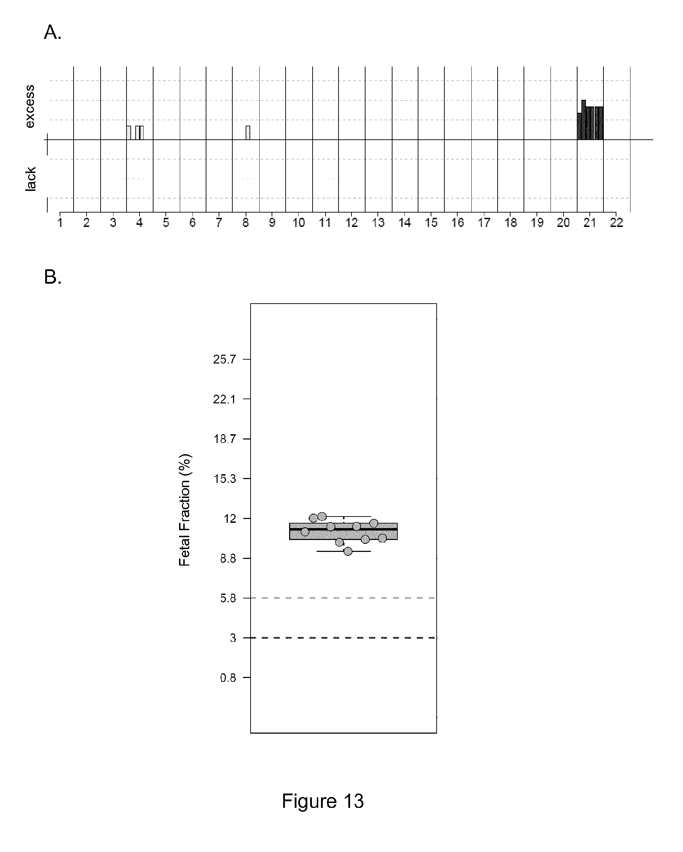

**Figure 13:** Example of a discrepancy (absence of correlation) between the aneuploidy signal intensity and the signal intensity (sample DPA-4670).

13A: Result of an aneuploidy test, for all autosomes (representation similar to Figure 9C). The lane 'chromosome 21" reveals a trisomy 21, as evidenced by an excess for all 6 comparisons with 6 reference sets of samples. The signal intensity is however low (the 6 signals are between the 1/1'000 and the 1/1'000'000 thresholds).

13B: determination of the fetal fraction (mean of 10 determinations). The lower dotted line represents the fetal fraction threshold R of about 3%, which is equivalent to the lowest-validated chromosomal dosage in a given reference set. Samples with a fetal fraction above the threshold R are considered as having a sufficient fetal fraction for a reliable diagnosis. The upper dotted line represents the fetal fraction of 5.8%. This threshold includes about 10% of the lowest fetal fractions. The estimated fetal fraction for the tested sample is 10.9 $\pm$ 0.9 %, which ranges this sample as an "average" sample, in terms of fetal fraction. Overall, there is a clear discrepancy between the low signal intensity and the average fetal fraction.

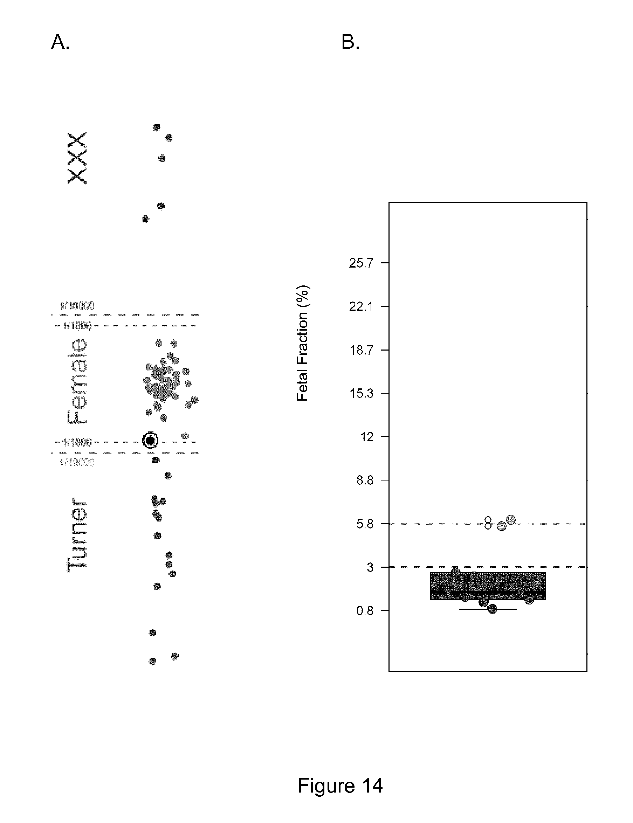

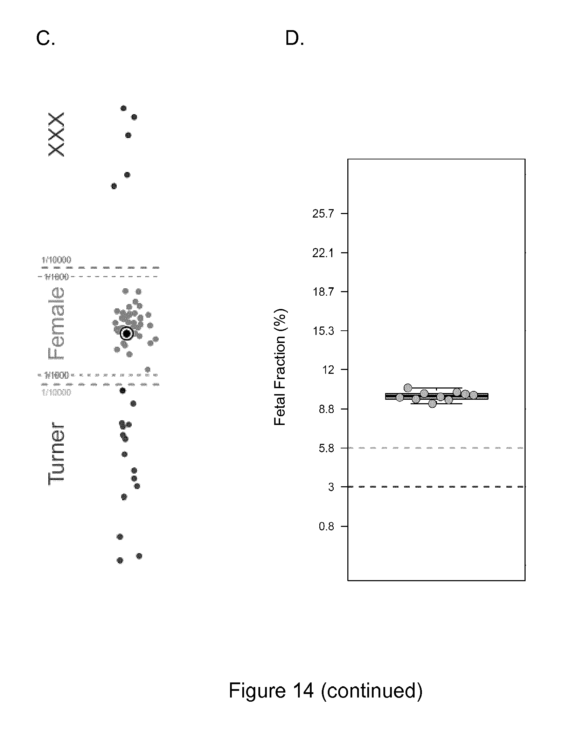

**Figure 14:** Enrichment of the fetal fraction by a second blood draw

14A and 14B: first blood draw. The sample is diagnosed euploid for chromosome X (14A). The fetal fraction is 2,5 $\pm$ 1,9%, i.e. lower than the threshold of 3% (14B).

14B: second blood draw, two weeks later. The fetal fraction is enriched (9,8 $\pm$ 0,4%, 14D). The sample is still diagnosed euploid for chromosome X (14C).

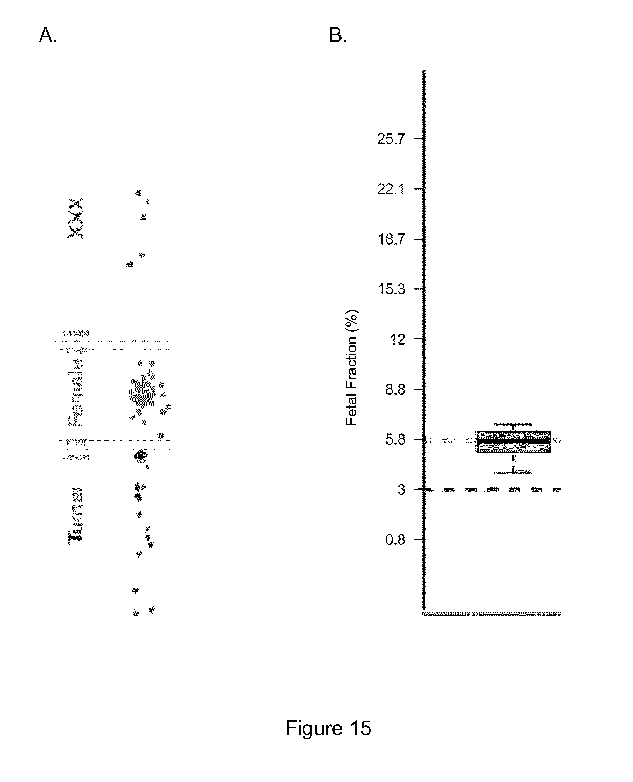

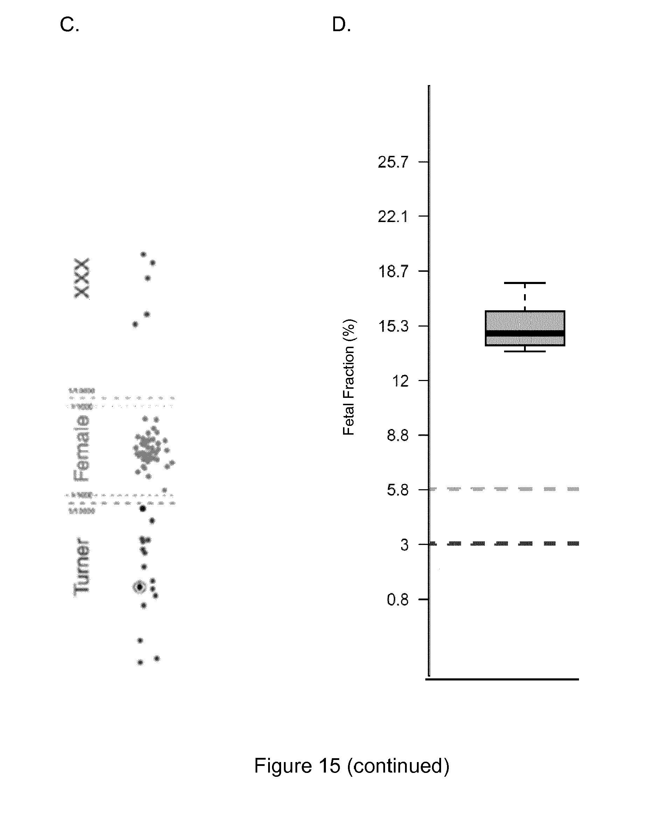

**Figure 15:** Enrichment of the fetal fraction by a second blood draw

15A and 15B: first blood draw. The sample is diagnosed as affected from a monosomy X, but the signal strength is close to the discrimination threshold (15A). The fetal fraction is. higher than the threshold of 3%, but still in a low range, in comparison with the average values (15B).

15C and 15D: second blood draw, two weeks later. The fetal fraction is enriched. The diagnosis of monosomy X is confirmed by the increase of the signal intensity (15C).

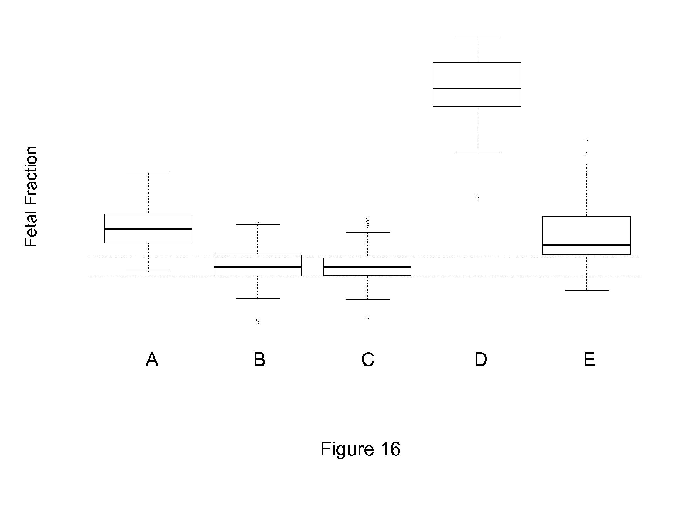

**Figure 16:** Enrichment of the fetal fraction by size selection. One protocol of manual DNA extraction (A) and four protocols of automatic DNA extraction (B to E) were applied to a blood sample. Protocol D was the only one to include a process of gel electrophoresis-based size selection, to eliminate DNA fragments with a size over 150 nucleotides. As evident from a comparison of protocol D with either of protocols B, C or E, size selection enriches the fetal fraction by 3-5 fold. It also enriches the fetal fraction at a higher level than that resulting from manual DNA extraction. The lower dotted line represents the fetal fraction threshold R of 3%, which is equivalent to the lowest-validated chromosomal dosage in a given reference set. The upper dotted line represents the fetal fraction of 5.8%, which includes about 10% of the lowest fetal fractions.

Definitions

Unless otherwise indicated by a statement, or by the context, the following terms have the meaning indicated below.

The term "**nucleic acid**" refers to a molecule composed of a chain of nucleotides. The term may refer to nucleotide chains of any composition, such as deoxyribonucleic acid (DNA), ribonucleic acid (RNA), RNA/DNA hybrids, DNA or RNA analogs or their hybrids, and /or polyamide nucleic acids (PNA). DNA may be complementary DNA (cDNA) and/or genomic DNA (gDNA). RNA may be messenger RNA (mRNA), short inhibitory RNA (siRNA), ribosomal RNA (rRNA), transfer RNA (tRNA) and/or microRNA. DNA or RNA analogs include base analogs, sugar analogs and/or analogs comprising a non-native backbone.

A nucleic acid may be single-stranded, double-stranded or partially single-stranded and double-stranded.

The term "**nucleic acid species**" refers to a group of nucleic acid molecules characterized by one or more common features, such as a common sequence, a common epigenetic state, and/or a common origin. A common sequence includes one or more sequence variations with respect to a reference sequence, such as one or more single nucleotide polymorphisms (SNP), additions, insertions, and/or deletions. An epigenetic state includes any structural feature at a molecular level of a nucleic acid, such as its secondary or tertiary structure, its methylation state, the presence and/or amount of one more particular sequences, such as CpG sites, tandem repeat sequences, inverted terminal repeat sequences, and/or palindromic sequences. A common origin of the nucleic acids can be a same chromosome or a same chromosomal region, for example a same region of 1, 2, 5, 10, 20, 50, 100, 200, 500 kilobases (kb), 1, 2, 5, 10, 20, 50, 100, 200, 500, 1000 Mb (megabases) on a chromosome. Alternatively, a common origin can mean that the nucleic acids originate from the same cell, cell type, tissue, organ and/or organism (such as an individual). For example, a nucleic acid species can comprise or consist of nucleic acids originating from a group of cells affected by a biological condition, such as a diseased tissue, for instance a tumor tissue. Alternatively, the nucleic acids from a nucleic acid species can derive from a group of cells, e.g. a tissue, not affected by a biological condition, for example from all cells in an organism except those originating from a particular diseased tissue. The nucleic acids from a nucleic acid species can also comprise or consist of nucleic acids deriving from an individual, such as a fetus or a pregnant woman. All the above criteria can be combined to define a set of common features shared by the nucleic acid molecules of a nucleic acid species, for example nucleic acids originating from the same chromosome of an individual, such as a fetus or a pregnant woman, or nucleic acids sharing a same sequence variation and a common origin, such as from a tumor cell.

When the present application refers to two different nucleic acid species or more (for example N1 and N2), it is meant that the two nucleic acid species or more differ by at least one feature, such as a sequence, an epigenetic state, and/or an origin. The differentiating feature can be selected in the same list as the common feature referred to in the preceding section. For example, a first nucleic acid species (N1) is derived from a fetus, whilst a second nucleic species (N2) is derived from the pregnant woman carrying the fetus. Alternatively, a first nucleic acid species (N1) is derived from a tumor tissue and/or a tumor cell, whilst a second nucleic acid species (N2) is derived from a non-tumor tissue and/or a non-tumor cell.

The terms "**derives from**", "**is derived from**", "**originates from**" or the likes in phrases such as "a nucleic acid derives from an individual" or "a nucleic acid derives from a cell/cell type/tissue", refer to the site of biosynthesis or biological origin of the nucleic acid. For example, a nucleic acid derived from a fetus (also referred to as a fetal nucleic acid) is a nucleic acid which has been synthesized in fetal cells, and a nucleic acid derived from a tumor (also referred to as a tumor nucleic acid) is a nucleic acid which has been synthesized in tumor cells.

The terms "**cell free nucleic acids**" or "**extracellular nucleic acids**" can be used interchangeably and refer to nucleic acids which circulate outside of cells, in bodily fluids, such as blood, blood plasma, blood serum, urine, saliva, and/or extracellular fluid. Without being limited by theory, extracellular nucleic acids are assumed to be a product of cell apoptosis, cell necrosis and/or cell breakdown. Cell free nucleic acids can be in the form of nucleic acid fragments having a length which can vary across a large spectrum.

By way of contrast, the terms "**cell nucleic acids**", "**cellular nucleic acids**" or "**intracellular nucleic acids**" designate nucleic acids contained within a cell. The isolation of these nucleic acids requires that the cell be degraded. The cellular nucleic acids may be nuclear nucleic acids, cytoplasmic nucleic acids and/or mitochondrial nucleic acids. Non-limiting examples of sources for cellular nucleic acids are blood cells, tissues cells, organ cells, tumor cells, hair cells, skin cells, or bone cells. In some embodiments, the cellular nucleic acids are from placental cells. In some embodiment, the nucleic acids are derived from the fetal part of the placenta. In some embodiments, nucleic acids are derived from the maternal part of the placenta.

The **"relative amount N1:N2"** is a parameter reflecting the comparison between two amounts: the amount of nucleic acid molecules or sequences from a nucleic acid species N1 and the amount of nucleic acid molecules or sequences from a nucleic acid species N2. Non-limiting examples of parameters representative of the relative amount N1:N2 are the ratio [N1]/[N2], the ratio [N1]/([N1]+[N2]), the difference [N1]-[N2], the weighted differences ([N1]-[N2])/([N1]+[N2]), ([N1]-[N2])/[N1] or ([N1]-[N2])/[N2], where [N1] and [N2] are respectively indicative of the amount of nucleic acids from the nucleic acid species N1 and N2. The ratios [N1]/[N2] or [N1]/[N1+N2] are preferred to represent the relative amount N1:N2. The ratio [N1]/[N1+N2] is particularly preferred, and represents the proportion of nucleic acids of the species N1, within the total pool of nucleic acids N1 + N2. Alternatively, the relative amount N1:N2 is a parameter derived from the parameters listed above, for example by one or several mathematical operations such as addition, division, multiplication, subtraction, or by statistical operations, provided that the derived parameter still reflects a comparison between the amount of nucleic acid molecules from a nucleic acid species N1 and the amount of nucleic acids from a nucleic acid species N2.

The **"total amount [N1+N2]"** is a parameter, or a set of parameters, reflecting the total count of molecules or sequences from nucleic acid species N1 and N2.

The **"fetal fraction"** is the ratio of the amount of cell-free fetal DNA to the total amount of cell-free DNA in a biological sample, in particular in a blood sample, from a pregnant woman. The total amount of cell-free DNA generally corresponds to cell-free (fetal + maternal) DNA. The fetal fraction is an example of relative amount N1:N2, in the case where the nucleic acid species N1 is cell-free fetal DNA (in particular originating from a particular chromosome/chromosomal region), and the nucleic acid species N2 is cell-free maternal DNA (in particular originating from a particular chromosome/chromosomal region). By extension, the term "fetal fraction" can be used in specific contexts to designate the ratio of the amount of cell-free fetal nucleic acids to the total amount of cell-free nucleic acids in a biological sample, in particular in a blood sample, from a pregnant woman.

As used herein the term **aneuploidy** refers to the variation of a quantitative amount of one chromosome from that of a diploid genome. The variation may be a gain, or a loss. It may involve a whole chromosome or a part thereof, for example only a chromosomal region. Aneuploidy can include monosomy (lack of one chromosome), partial monosomy (translocation or deletion of a portion of a chromosome), trisomy (gain of one extra chromosome), partial trisomy (gain and/or duplication of a portion of a chromosome). **Euploidy** is herein used to mean the contrary of aneuploidy. A euploid sample thus refers to a diploid genome, chromosome or chromosomal portion. For instance, an individual euploid for chromosome 21 has two copies of the chromosome 21.

The term **"statistically significant"** means that an observation or an event is not attributed to random chance. "Statistically significant" may be characterized by a p value of less than 0.05, or less than 0.01.

The **"test sample"** or **"test biological sample"** is the sample being tested, as opposed to the **"reference sample(s)"** or **"reference biological sample(s),** which are used as a basis for comparing the relative amount N1:N2, the total amount [N1+N2], or any other parameter derivable from the test sample.

The term **"specific"** as in "a discrimination threshold R is specific for the method of step (a)" means that the discrimination threshold is specially adapted to the method of step (a), or, in other terms, it takes into account the specificities of method (a). A specific threshold is opposed to a threshold defined, e.g. theoretically, without considering the specificities of method (a). A specific discrimination threshold R is defined, for instance, by implementing the method of step (a) on one or more reference samples, and defining the discrimination threshold R from a parameter of the reference sample(s), in particular the ratio N1:N2 in the reference sample(s).

## Detailed description

One aspect of the present invention is a method for determining the presence of a biological condition from a biological sample.

### Biological sample

The method according to the present invention can be carried out on any biological sample containing nucleic acids. The method is preferably an *in vitro* method, the biological sample having been removed from the body. The biological sample is for example derived from a bodily fluid, a tissue, or an organ. In one embodiment, the biological sample contains cells from the patient. In another embodiment, the biological sample is essentially cell-free. Where the biological sample is derived from a bodily fluid, the bodily fluid can be blood, blood plasma, blood serum, urine, breast milk, saliva, amniotic fluid, cerebrospinal fluid (CSF), mucus, peritoneal fluid, pleural fluid, synovial fluid. Blood, blood plasma or blood serum are preferred, blood plasma being particularly preferred. Where the biological sample is derived from a tissue or an organ, it is for example derived from a diseased tissue or organ, such as a tissue or organ affected by a tumor. The biological sample can be derived from an invasive procedure, such as a biopsy, chorionic villus sampling, amniocentesis, or from a non-invasive procedure such as blood sampling, urine sampling, CSF sampling, breast milk sampling, mucus sampling, or peritoneal fluid sampling.

The biological sample can originate from any mammalian, preferably a human patient, including a pregnant woman, a patient with a predisposition to a biological condition, such as cancer, or a patient affected from biological condition, such as cancer. The biological sample can also originate from a patient with no known predisposition to the biological condition to be detected and/or a patient not affected by said biological condition. Where the patient is a pregnant woman, the objective of the method of determining the presence of a biological condition may be prenatal testing, i.e. the detection of a biological condition of the fetus carried by the pregnant woman. In such a case, the biological samples can be collected at any time during the pregnancy, but are preferably collected from 7 weeks of pregnancy, for example between 7 weeks and 20 weeks of pregnancy, preferably from 7 to 14 weeks of pregnancy, still preferably from 7 to 12 or 7 to 10 weeks of pregnancy. A prenatal testing performed as early as 7 to 12 weeks of pregnancy provides the advantage of keeping more medical options opened in cases where a decision to interrupt the pregnancy may need to be taken (for example, an interruption through the use of a drug or a combination of drugs may be allowed depending on the national laws). It also allows information to be given to the parents as soon as possible so that they can prepare the arrival of their baby in good conditions, for example by offering them psychological support. Where the biological samples are collected from a pregnant woman, through a non-invasive procedure, they can be collected following an invasive prenatal procedure, such as chorionic villus sampling, amniocentesis, or cord blood sampling. The biological samples can be collected at any time following the invasive procedure, for example at least 10 min, 20 minutes or 30 minutes following the invasive procedure. The biological samples can also be collected at least one or more days following the invasive procedure, for example from two to five days following the invasive procedure. Alternatively, the biological samples can be collected from women not yet having experienced an invasive prenatal procedure.

The biological sample generally contains nucleic acids, such as DNA and/or RNA. The nucleic acids can originate from the patient himself or herself and/or from a patient's fetus and/or from another organism, such as a virus, a bacteria, a fungus and/or a parasite. In one embodiment, the biological sample contains cell-free nucleic acids, in particular cell-free DNA and/or cell-free RNA.

Two nucleic acid species N1 and N2 are present in the biological sample. Various criteria can underlie the distinction between the two nucleic acid species, for example their origin, their epigenetic state, their biochemical nature, their sequence, their composition, or a combination of these criteria. In one embodiment, the two nucleic acid species N1 and N2 are respectively fetal cell-free nucleic acids (e.g. DNA or RNA) and maternal cell-free nucleic acids (e.g. DNA or RNA). In another embodiment, the two nucleic acid species are respectively tumor cell-free nucleic acids (e.g. DNA or RNA) and non-tumor cell-free nucleic acids (e.g. DNA or RNA). In a preferred embodiment, N1 and N2 share at least one common characteristic, for example common sequences, but can be distinguished on the basis of at least one further characteristic, for example, their size. Step (a) of the method according to the invention normally relies on the common characteristic, whereas step (b) relies on the distinguishing characteristic.

Biological condition

The biological condition can be any biological condition associated with a nucleic acid species N1. The term "associated" includes any relation, either proved or hypothesized, between the biological condition and the nucleic acid species N1. It can imply, but does not necessarily imply, a causative link between the biological condition and the nucleic acid species N1.

In one embodiment, the presence of a causative link between N1 and the biological condition means that the nucleic acid species N1 is a cause of the biological condition. The nucleic acid species N1 can be the sole cause for the biological condition, for example in the case of a genetic condition caused by the nucleic acid species N1. Alternatively, there can be several causes for the biological condition, including causes not related to the nucleic species N1. The other causes may be related to other nucleic acid species, and/or may have a non-genetic origin, such as an environmental origin.

In an alternate embodiment, the biological condition is the cause of the presence of the nucleic acid species N1. The nucleic acid species N1 is for example a marker of the biological condition, i.e. a molecule species which indicates the presence or a predisposition to the biological condition.

The biological condition may be a disease, a predisposition to a disease, a degree or severity of a disease. In one embodiment, the biological condition is a genetic condition, such as a genetic disorder. The genetic condition can be heritable or non-heritable.

In another embodiment, the biological condition is a non-genetic condition.

In one embodiment, the biological condition is a copy number variation, also referred to as aneuploidy. The copy number variation can be a germline copy number variation. In another embodiment, it is a somatic copy number variation. The copy number variation can be a chromosomal aneuploidy, or a subchromosomal copy number variation. Aneuploidy can include monosomy (lack of one chromosome), partial monosomy (translocation or deletion of a portion of a chromosome), trisomy (gain of one extra chromosome), partial trisomy (gain and/or duplication of a portion of a chromosome).

Examples of monosomy or partial monosomy include Wolf-Hirschhorn syndrome, cri du chat syndrome, 5q deletion syndrome, Williams syndrome, Jacobsen syndrome, Angelman syndrome, Prader-Willi syndrome, Miller-Dieker syndrome, Smith-Magenis syndrome, 18q deletion syndrome, DiGeorge syndrome.

Examples of trisomy include trisomy 1, trisomy 2, trisomy 3, trisomy 4, trisomy 5, trisomy 6, trisomy 7, trisomy 8 (Warkany syndrome), trisomy 9, trisomy 10, trisomy 11, trisomy 12, trisomy 13 (Patau syndrome), trisomy 14, trisomy 15, trisomy 16, trisomy 17, trisomy 18 (Edwards syndrome), trisomy 19, trisomy 20, trisomy 21 (Down syndrome), trisomy 22.

Other examples of disorders involving a loss (deletion) of one or several chromosomal regions include 1p36 deletion syndrome, TAR deletion, 1q21.1 deletion, 2q11.2 deletion, 2q11.2q13 deletion, 2q13 deletion, 2q37 deletion, 3q29 deletion, Wolf-Hirschhorn deletion, Sotos syndrome deletion, 6q16 deletion, Williams syndrome deletion , WBS-distal deletion, 8p23.1 deletion, 9q34 deletion, 10q23 deletion, Potocki-Shaffer syndrome, SHANK2 FGFs deletion, 12q14 deletion syndrome, 13q12 deletion, 15q11.2 deletion, Prader-Willi/Angelman syndrome, 15q13.3 deletion, 15q24 BP0-BP1 deletion, 15q24 BP0-BP1 deletion, 15q24 BP2-BP3 deletion, 15q25.2 deletion, Rubinstein-Taybi syndrome, 16p13.11 deletion, 16p11.2p12.1 deletion, 16p12.1 deletion, 16p11.2 distal deletion, 16p11.2 deletion, 17p13.3 deletion, 17p13.3 deletion, HNPP, Smith-Magenis syndrome deletion, NF1 deletion syndrome, RCAD (renal cysts and diabetes), 17q21.31 deletion, DiGeorge/VCFS deletion, 22q11.2 distal deletion, Phelan-McDermid syndrome.

Examples of disorders involving the duplication of a chromosomal region include the split hand/split foot syndrome 3, the Beckwith-Wiedemann Syndrome, the Pelizaeus-Merzbacher disease, the Charcot-Marie-Tooth disease type 1A, 15q duplication-related autism.

In another embodiment, the biological condition is pre-eclampsia.

In another embodiment, the biological condition is cancer, in particular bladder cancer, breast cancer, cervical cancer, colorectal cancer, hepatocellular carcinoma, lung cancer, lymphoma, such as Non-Hodgkin's lymphoma, melanoma, ovarian cancer, pancreatic cancer, prostate cancer, nasopharyngeal cancer, testicular cancer, oesophageal cancer, uterine cancer, liposarcoma, adenocarcinoma, gastric cancer, leiomyosarcoma, mesothelioma, glioma, renal cancer, neuroblastoma, medulloblastoma, synovial sarcoma, or acute lymphoblastic leukemia.

As used herein, determining the presence a biological condition includes, but is not limited to determining the presence of a disease, a predisposition to a disease, the intensity, severity and/or degree of a disease and/or the probability of presence or predisposition to a disease. The determination may be in a quantitative way and/or in a qualitative way. Hence, the outcome of the determination can be "positive" or "negative", but it can also be "indeterminate" where the method does not allow the drawing of a conclusion on the presence, predisposition, intensity or whatever parameter looked after. The determination can also result in a qualitative probabilistic appreciation, such as "probable", "highly probable", "low probability", etc...

In one embodiment, the present method for determining the presence of a biological condition in a biological sample comprises three steps:

a) determining the total amount [N1+N2] and comparing said total amount to at least one discrimination threshold T;

b) determining the relative amount N1:N2 and comparing said relative amount to at least one discrimination threshold R specific for the method of step (a); and

c) on the basis of the comparisons carried out in step (a) and (b), determining the presence of the biological condition.

Steps a) and b) can be carried out in either order.

## Total amount [N1 + N2]

Step (a) of the method for determining the presence of a biological condition according to the invention involves the comparison of the total amount [N1 + N2] of two nucleic acid species N1 and N2 within the biological sample, to a discrimination threshold T.

In one embodiment, the method according to the invention involves, before the comparison, the determination of the total amount [N1 + N2] of two nucleic acid species N1 and N2 within the biological sample. N1 and N2 are preferably determined concurrently. Specifically, the total amount [N1 + N2] is preferably determined without distinguishing between nucleic acid species N1 and N2. This may be due to the fact that N1 and N2 are indistinguishable by certain of their features, either generally, or in the particular method used to determine the total amount [N1 + N2]. For example N1 and N2 can share common sequences, and/or share a common locus on the genome (in the case of sequence variants, such as SNPs), and/or a common size or size distribution, and/or a common epigenetic state, such as a secondary or tertiary structure, a methylation pattern, a proportion of certain sequences, for example CpG sites. In a preferred embodiment, the determination of the total amount of [N1 + N2] is made on the basis of one or more common features of N1 and N2, such that a distinction between N1 and N2 cannot be made.

For example, the determination of the total amount [N1 + N2] is done by counting the sequences aligning uniquely, without mismatch, to a reference genome, or a portion thereof. In this way, the sequences shared by the nucleic acid species N1 and N2 are not distinguishable.

The determination of the total amount [N1+N2] can include various procedures of molecular biology generally applicable to nucleic acids, for example PCR, such as digital PCR, quantitative PCR (qPCR), reverse transcription PCR (RT-PCR) reverse transcription quantitative PCR (RT-qPCR), nested PCR, multiplex PCR, methylation-specific PCR, allele-specific PCR, emulsion PCR, hybridization, such as southern blotting, hybridization on a nucleic acid-array, e.g. a DNA-array, sequencing, chromatography, such as affinity chromatography, gel permeation chromatography, liquid chromatography, including high performance liquid chromatography (HPLC), or any combination of these techniques.

In one embodiment, the method according to the invention comprises the extraction of the nucleic acids from the biological sample, for example on a solid support such as beads, e.g. by magnetic beads, or in a liquid medium, such as an alcohol-containing and/or chloroform-containing solution, e.g. a phenol and chloroform-containing solution. The extraction may involve the use of an extraction column.

In one embodiment, the method according to the invention comprises a step of sequencing. The sequencing may be carried out on the whole genome, whereas in other embodiments, it concerns only a portion of the genome.

Although various methods of sequencing can be contemplated, high throughput methods are preferred, such as massively parallel sequencing (MPS), also referred to as next-generation sequencing (NGS) or second-generation sequencing (Buermans et al., 2014).

The massively parallel sequencing technologies generally comprise two main steps: the preparation of a set of templates (sequencing library), on which the actual sequencing will be carried out, and the actual step of sequencing, which generally comprises the detection of the addition or release of nucleotides on the template.

The preparation of the sequencing library can take place immediately after the extraction of the nucleic acids or it can take place after a prior processing of the extracted nucleic acid. The prior processing is for instance an enrichment of a particular nucleic acid species, such as a size-selection of the extracted nucleic acids.

The preparation of the sequencing library can include one or more amplification steps, a ligation with one or more sequencing adaptors, and/or barcoding the nucleic acid molecules. A typical workflow of the sequencing library preparation includes a step of ligation of one or more adaptor sequences, optionally linked to one or more barcode sequences, to the nucleic acid molecules inside the sample, followed by an amplification of the adaptor/barcode-ligated nucleic acid molecules.

Sequencing adaptors are short nucleotide sequences which are commonly used in modern sequencing technologies. The adaptors are used for anchoring the nucleic acid molecules to be sequenced to a solid surface, for example in a flow cell. These adaptors are thus designed so as to hybridize to target oligonucleotides tethered to the solid surface. The ligation of adaptors is preferably performed by repairing the ends of the nucleic acid molecules, i.e. suppressing or filling out the overhangs of the extracted nucleic acid molecules, for example through the action of one or more exonucleases and/or polymerases, thus yielding blunt-ended nucleic acid molecules. An overhang of one or more 'A' bases may then be optionally added at the 3' end of the blunt-ended nucleic acid molecules. The adaptors containing an overhang of one or more 'T' bases at their 3' end, are then added and are ligated to the overhang of one or more 'A' bases at the 3'end of the nucleic acid molecules. Adaptors can also be blunt ligated.

The nucleic acid molecules within the sample can also be barcoded. Barcoding refers to the ligation of a sample-specific tag to the nucleic acid molecules of a sample. Barcoding allows the sequencing of several samples in a single sequencing run, which saves time and resources.

The nucleic acid molecules within the sample can also be subjected to one or more amplification cycles, for example by PCR. The amplification can be performed by bridge amplification, a technique which uses templates attached on a solid support. It can also be performed by emulsion PCR. The amplification is preferably carried out after the ligation of an adaptor sequence to the nucleic acid molecules. The PCR amplification preferably uses primers against the adaptor sequence, thus enriching the library into adaptor-ligated fragments. In some cases, amplification is not necessary, in particular in sequencing platforms with a detection limit sufficient to detect a single nucleic acid fragment.

The sequencing is then carried out on the sequencing library, generally by detecting the addition/release of nucleotides on the sequencing templates. Several technologies exist for the detection, such as visible/UV light detection, fluorescence detection, or pH detection, i.e. detection of the addition or release of a proton ($H^+$) in the reaction medium.

Anyone of the NGS platforms that are available commercially, are currently under development, or will be developed in the future, can be used in the present invention, for example the platforms GS FLX Titanium XL+ (Roche), GS Junior System (Roche), 454 (Roche), Ion Torrent (Life Technologies), Proton (Life technologies), Abi/solid (Life technologies), HiSeq2000/2500 (Illumina), MiSeq (Illumina), NextSeq500 (Illumina), HiSeq X Ten (Illumina), RSII (Pacific biosciences) or Heliscope (Helicos).

The massively parallel sequencing platform consists for instance in a "sequencing-by-synthesis" system, such as the Illumina's HiSeq2000 or HiSeq2500 platforms. These platforms use a reversible terminator-based method that detects single bases as they are incorporated into growing DNA strands. The sequencing workflow can be summarized in 3 phases:

- First, the preparation of the DNA library. During this phase, DNA molecules are ligated with adaptors at both ends. In addition, they contain primer sites that are used to amplify the library by PCR and to sequence it.

- Second, the cluster generation: during this phase, DNA molecules are hybridized to oligonucleotide probes tethered on a solid surface inside a flow cell. Each DNA molecule is amplified by solid-phase bridge-amplification, forming a cluster of molecules with identical sequences.

- Third, the "sequencing-by-synthesis" phase. A mixture of the four nucleotides, each containing a fluorescently-labeled terminator, is introduced into the flow-cell. The fluorescently-labeled terminator is imaged as each dNTP is incorporated into the growing DNA strand, and then cleaved to allow incorporation of the next base. Since all four reversible terminator-bound dNTPs are present during each sequencing cycle, natural competition minimizes incorporation bias. Base calls are made directly from intensity signal measurements during each cycle.

Alternatively, the massively parallel sequencing platform may consist in a semiconductor-based NGS technology, such as the Ion torrent technology developed by Life Technologies. In this platform, the sequence templates are generated on a bead or sphere via emulsion PCR. The loaded spheres are than deposited on a sequencing chip consisting of a flow compartment and solid state pH sensor micro-arrayed wells that are manufactured using processes built on standard CMOS technology. The detection of the incorporated bases during sequencing is not based on imaging of fluorescent signals, but on the release of a proton ($H^+$) during extension of each nucleotide. The release of the proton is detected as a change in the pH within the sensor wells. Since there is no detectable difference for a proton released from an A, C, G or T bases, the individual dNTPs are applied in multiple cycles of consecutive order. If upon delivery of a dNTP, a pH change is detected, this base is in the template. The intensity of the pH change will indicate a repeated base, i.e. a series of the same nucleotide.

In a specific embodiment, the sequencing step yields at least 10 million sequences per sample, or at least 15 million, 20 million, 25 million or 30 million sequences per sample.

The sequences obtained by sequencing can be mapped to a reference sequence, or in other terms aligned with a reference sequence. "Indexing" is also used as a synonym of "mapping", in the present disclosure. The alignment of the sequences can be carried out using any standard alignment software, for example as described in Chiu et al., 2008 or Fan et al., 2008. The reference sequence is preferably a reference sequence of the human genome, such as the sequences established by the NBCI (http://www.ncbi.nlm.nih.gov/assembly/2758/) or the UCSC (http://hgdownload.cse.ucsc.edu/downloads.html#human). The reference sequence is preferably the Genome Reference Consortium GRCh37 released in February 2009, also referred to as hg19; or the Genome Reference Consortium GRCh38 released in December 2013, also referred to as hg38.

Mapping can be done over the whole genome, or over only a portion of the genome.

Generally speaking, a partial sequence of a genome, such as the human genome used in score calculation is obtained by masking predefined regions of the genome. The regions to be masked can be chosen on the basis of a number of different parameters, including: a lower quality of sequencing of a region (these regions are also known as "non-well annotated regions"); the occurrence of a high number of repeats within a region; the duplication of a region within the genome; a region with a complex architecture. The masked regions are thus preferably selected among the non-well-annotated regions of the genome, the high copy repeat regions of the genome, the duplicated regions of the genome, or the regions with a complex architecture.

A region with a lower quality of sequencing or a "non-well annotated" region is for instance a region with scaffold N50 of less than 46,395,641 and/or a contig N50 of less than 38,508,932, and/or with total assembly gap length of more than 239,845,127/3,137,144,693, and/or with a genome coverage of at least 90%, preferably at least 95% (Yandell et al., 2012). Examples of non-well annotated regions are subtelomeric regions and pericentromeric regions.

Genome assemblies are composed of scaffolds and contigs. Contigs are contiguous consensus sequences that are derived from collections of overlapping reads. Scaffolds are ordered and orientated sets of contigs that are linked to one another by mate pairs of sequencing reads. A contig N50 is calculated by first ordering every contig by length from longest to shortest. Next, starting from the longest contig, the lengths of each contig are summed, until this running sum equals one-half of the total length of all contigs in the assembly. The contig N50 of the assembly is the length of the shortest contig in this list. The scaffold N50 is calculated in the same way but uses scaffolds rather than contigs. Scaffolds and contigs that comprise only a single read or read pair - often termed 'singletons' - may be excluded from these calculations, as may be contigs and scaffolds that are shorter than -800 bp.

Genome coverage refers to the percentage of the genome that is contained in the assembly based on size estimates; these are usually based on cytological techniques. A region with a complex architecture is for instance a highly variant region, for example a region with a high number of CNVs (copy number variants), and/or SNVs (single nucleotide variants) (Frazer et al., 2009). An estimate of 5% of the human genome is for instance copy number variable.

The sequences are then counted from the mapping step. This is generally done by counting certain categories of sequences.

Among the sequences which can be counted from the mapping are the Unique Exact Sequences (UES) or Unique Exact Matches (UEM), which are sequences which are uniquely mapped to the reference sequence with no mismatch. By 'unique' or 'uniquely', it is meant that the sequence has a single match on the reference sequence, i.e. it does not match to different genome regions. In a specific embodiment, at least 6 million, preferably at least 8 million, still preferably at least 10 million, or at least 12 million or at least 14 million or at least 15 million unique exact sequences per sample are obtained in the mapping step.

Other categories of sequences can be counted/highlighted from the mapping steps, such as unique sequences with at most one, or two or three mismatches, or aligned exact sequences (unique or not unique), or aligned sequences with at most one, or two or three mismatch, or aligned sequences with at most 1%, 2%, 5%, 10%, 15%, 20% mismatched nucleotides.

Calculation of a score representing the total amount [N1 + N2]

Step (a) of comparison of the total amount [N1 + N2] with a discrimination threshold T is not necessarily achieved by a direct comparison of the total amount [N1 + N2] to the discrimination threshold T, but can be carried out through the bias of one or several parameters, or 'scores' indicative of the total amount [N1 + N2]. Where the method according to the invention comprises or is associated with sequencing and mapping steps, the total amount [N1 + N2] can be represented by a 'score' calculated for a given chromosome or chromosomal region, i.e. a parameter indicative of the count of sequences mapped to a chromosome or chromosomal region. Such a score can for instance be calculated in the case of a detection of an aneuploidy, through the analysis of cell free maternal and fetal nucleic acids.

In one embodiment, the score is calculated from the UEMs. In another embodiment, the score is calculated from the unique sequences with at most one, or two, or three mismatches. In another embodiment, the counted sequences are the aligned exact sequences (unique and not unique), and/or the aligned sequences with at most one, or two or three mismatch, or the aligned sequences with at most 1%, 2%, 5%, 10%, 15%, 20% mismatched nucleotides. The score can also be calculated from any combination of these sequence categories.

The score can be calculated over a whole genome sequence, or over a partial sequence of a genome or, in other terms a sequence from which some regions have been masked. Calculating the score only over a carefully selected portion of a genome is a way to increase the degree of statistical confidence of the diagnosis method. Generally speaking, the partial sequence of the genome used in score calculation is obtained by masking predefined regions of the human genome, in the same way at that explained previously to obtain a partial sequence of the genome. Accordingly, the same parameters can be considered for defining the regions to be masked, including a lower quality of sequencing of a region (also defined, in other terms as a non-well annotated region), the occurrence of a high number of repeat within a region, the duplication of a region within the human genome, a region with a complex architecture. The masked regions are thus preferably selected among the non-well-annotated regions of the genome, the high copy repeat regions of the genome, the duplicated regions of the genome or the regions with a complex architecture.

The score for each chromosome can be calculated by dividing each chromosome into bins of a predefined length, for example bins with a length of at least 10kb, 20kb, 50 kb, 100 kb, 200 kb, 500 kb, 1 Mb, 2 Mb, 5 Mb or 10 Mb.

The number of sequences mapped to a given bin is then counted, thus yielding a sequence count for each bin.

In a specific embodiment, the count of sequences for each bin is bias-corrected, i.e. it is corrected to take into account the bias related to the sequencing process. A known bias is caused by the variation in GC distribution across the genome. Indeed, the distribution of sequence tags across the genome is not uniform (Fan and Quake, 2010). There is a positive correlation between the GC content of a chromosomal region, and the number of sequences mapped to said region, which explains why sequences originating from GC-rich regions are more represented within the sequence library than sequences originating from GC-poor regions. This bias can be compensated by weighting the count of sequences in each bin, for example with a weight inversely proportional to the GC content in said bin.

The median sequence count value for all bins over a chromosome or chromosomal region of interest can then be calculated. This value is representative of the count of sequences across the chromosome or chromosomal region, and is referred as the sequence tag density of a chromosome or chromosomal region. This median value can be calculated by using non-weighted sequence counts, or by weighting each sequence count with a bias-correction factor, such as a GC-bias correction factor, as indicated above. In another embodiment, other values than the median value are selected for representing the sequence count across a chromosome or chromosomal region: for instance the total sequence count for all bins within a chromosome or chromosomal region.

Finally, the sequence tag density of the chromosome or chromosomal region of interest can be normalized to the median sequence tag density for all chromosomes. Alternatively, it can be normalized to the median sequence tag density for all autosomes. Still alternatively, it can be normalized to the median sequence tag density for a predefined set of chromosomes. As used herein "set of chromosomes" refers to any combination of chromosomes selected from chromosome 1 to chromosome 22 and chromosome X and Y. Still alternatively, it can be normalized to the median sequence tag density for a predefined set of chromosomal regions. Still alternatively, it can be normalized to the sum of sequence tag densities for all chromosomes, or for all autosomes, or for all autosomes plus chromosome X or Y, or for a predefined set of chromosomes, or for a predefined set of chromosomal regions.

The normalized sequence tag density of a chromosome or chromosomal region can be used as a parameter indicative of the number of unique exact sequences mapped to a chromosome or chromosomal region for a given sample, and thereby as a "score". A number of other parameters can be used instead:

- The total count of sequences, such as UESs, mapped to a chromosome or chromosomal region.

- the sequence tag density of a chromosome or chromosomal region;

- the optionally GC-normalized count of sequences, such as UESs, mapped to said chromosome or chromosomal region;

- the optionally GC-normalized count of sequences, such as UESs, mapped to said chromosome or chromosomal region normalized by the total count of sequences, such as UESs, for the sample;

- the optionally GC-normalized count of sequences, such as UESs, mapped to said chromosome or chromosomal region normalized by the total number of sequences, such as UESs mapped to a predefined chromosomes or chromosomal regions set or to a predefined set of chromosomes or chromosomal regions.

In one embodiment, the chromosome of interest is chromosome 21 and/or the biological condition is fetal trisomy 21. Alternatively, the chromosome of interest is chromosome 18 and/or the biological condition is fetal trisomy 18. Alternatively, the chromosome of interest is chromosome 13 and/or the biological condition is fetal trisomy 13. Alternatively, the chromosome of interest is chromosome 22 and/or the biological condition is fetal trisomy 22. Alternatively, the chromosome of interest is chromosome 4 and/or the biological condition is fetal Wolf-Hirschhorn syndrome.

Alternatively, the chromosomal region of interest is a portion of chromosome 4 comprising the deleted region in Wolf-Hirschhorn syndrome. Alternatively, the chromosome of interest is chromosome 5 and/or the biological condition is fetal cri du chat syndrome. Alternatively, the chromosomal region of interest is a portion of chromosome 5 comprising the deleted and/or duplicated region in cri du chat syndrome and/or the biological condition is fetal cri du chat syndrome. Alternatively, the chromosome of interest is chromosome 19. Alternatively, the chromosome of interest is chromosome 1. Any combination of the aforementioned chromosomes or chromosomal region can also be chosen as a specific embodiment.

More preferably, the chromosome of interest is chromosome 21, chromosome 18, or chromosome 13, still preferably, the chromosome of interest is chromosome 21 or chromosome 18.

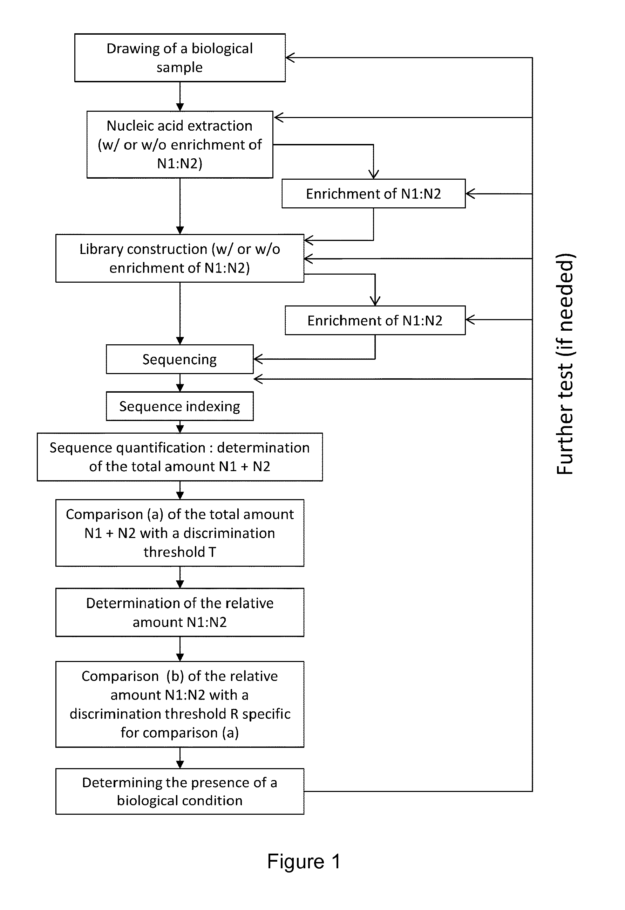

Copy number variations (CNVs) have been reported to be useful markers of cancer tissue, and are detectable from the plasma nucleic acids as well (Chan et al., 2013). Tumoral cell-free nucleic acids will contribute to increase the total amount of cell-free nucleic acids (tumoral and non-tumoral). Hence, a CNV will be detected through an increase of total cell-free nucleic acids, in the relevant chromosomal/chromosomal region. The CNVs can be detected in a genome-wide analysis, or a focused analysis limited to predefined regions. Hence, in one embodiment, the chromosome or chromosomal region of interest is a chromosome or chromosomal region which is subject to copy number variations in a cancer, in particular in a bladder cancer, breast cancer, cervical cancer, colorectal cancer, hepatocellular carcinoma, lung cancer, lymphoma, such as Non-Hodgkin's lymphoma, melanoma, ovarian cancer, pancreatic cancer, prostate cancer, nasopharyngeal cancer, testicular cancer, oesophageal cancer, uterine cancer, liposarcoma, adenocarcinoma, gastric cancer, leiomyosarcoma, mesothelioma, glioma, renal cancer, neuroblastoma, medulloblastoma, synovial sarcoma, or acute lymphoblastic leukemia. Chromosomes, chromosomal arms, chromosomal regions or genes subject to copy number variations in cancer, generally somatic copy-number variations, are for example described in Beroukhim et al., 2010, the disclosure of which is hereby entirely incorporated by reference (see e.g. the arms shown in Fig. 1b, the genes listed in Fig. 1c, and the regions listed in Supplementary Tables 2, 3 and 5 to 8).

Somatic copy-number variations have also been found to be involved in neuropsychiatric disorders (Cai et al., 2014).

Comparison of the total amount [N1 + N2] to at least one discrimination threshold T

The method according to the invention comprises a step (a) in which the total amount [N1 + N2] in the test biological sample is compared with one or several discrimination thresholds T. This step consists, for example, in comparing the total amount of fetal + maternal cell-free DNA from a chromosomal or chromosomal region, to at least one discrimination threshold T. In another embodiment, this steps consists in comparing the total amount of tumoral + non-tumoral cell-free DNA from a chromosomal or chromosomal region, to at least one discrimination threshold T. The discrimination threshold T can serve to establish a "first-level" determination, or "pre-determination" of whether the tested sample is positive or negative for the biological condition (i.e. a first level diagnosis, where the determination is a diagnosis). For example, where the detected condition is aneuploidy, this step is an aneuploidy calling, i.e. a "first-level" determination or "pre-determination" of whether the test is positive or negative for aneuploidy. In one embodiment, if the total amount [N1 + N2] is above the threshold T, the sample is assumed to be as positive for the biological condition. On the contrary, if the total amount [N1 + N2] is below the discrimination threshold, the sample is assumed to be negative for the biological condition. In both cases, the first-level determination carried out in step (a) needs to be confirmed by carrying out step (b), to verify that the relative amount N1:N2 is itself above a predefined threshold N1:N2, which will confirm the reliability of the first comparison (step (a)).

Alternatively or in addition, the discrimination threshold T serves to assess the strength/intensity of the signal for the total amount [N1 + N2]. The assessment may be quantitative and/or qualitative. The sample is for example rated as having a very low, low, moderate, high, or very high signal strength for the total amount [N1 + N2] on the basis of a comparison with one or more discrimination threshold(s) T.

In one embodiment, comparing the total amount [N1 + N2] to at least one discrimination threshold T comprises determining whether said total amount [N1 + N2] is above or below the discrimination threshold T. Alternatively, or in addition, comparing the total amount [N1 + N2] to at least one discrimination threshold T comprises assessing the distance between the total amount [N1 + N2] and the discrimination threshold T.

In one embodiment, the discrimination threshold T is internal to the biological sample, i.e. it is obtained from the analysis of the same test biological sample as that analyzed in the framework of the method for determining the presence of a biological condition. The discrimination threshold T is for example obtained from the analysis of further nucleic acid species in the test biological sample (in such a case, the further nucleic acid species are referred to as "reference nucleic acid species"). In one embodiment, N1 and N2 are nucleic acids from test chromosome(s) and/or chromosomal region(s), and the discrimination thresholds are obtained from the analysis of reference chromosomes or chromosomal regions different from the test chromosome(s) or chromosomal region(s). In one embodiment, the internal discrimination thresholds is obtained from nucleic acid species not related to the biological condition. For example, if the biological condition is a chromosome aneuploidy, the discrimination threshold may be obtained from one or more reference chromosomes not affected by the aneuploidy (e.g. any autosome but chromosome 21, in the case of trisomy 21 detection).

In another embodiment, the discrimination thresholds T are external to the test biological sample, i.e. they are obtained from one or more reference samples different from the test sample. The external discrimination thresholds are for example the total amount of the nucleic acid species [N1 + N2] in the reference samples, or any parameter indicative of this total amount.

The reference samples for the purpose of obtaining discrimination thresholds T are selected on the basis of various criteria, including the sample quality. Where the method involves massively parallel sequencing, the reference samples can be selected according to the number of total sequences obtained, and/or the number of UEMs. Exemplary criteria for selecting reference samples for the purpose of deriving discrimination thresholds T, in the field of NIPT, are described in the PCT publication WO2014/068075, the content of which is hereby incorporated by reference. In one embodiment, the reference samples are not affected by the biological condition. In another embodiment, the reference samples comprise both samples affected and non-affected the biological conditions, and are gathered into one or more sets of samples comprising both affected and non-affected samples.

In one embodiment, the comparison of the total amount [N1 + N2] with the discrimination threshold T is carried out by calculating the z-score of the test sample, according to the formula:

$$\text{z-score} = (P_{test} - \text{mean}\,(P_{ref}))/(SD(P_{ref}))$$

wherein

- $P_{test}$ is the score representing [N1 + N2], calculated from the test biological sample.

- Mean ($P_{ref}$) and SD($P_{ref}$) are respectively the mean and the standard deviation of the set of reference scores.

In this formula, the reference scores can be internal reference scores or external reference scores, such as reference scores obtained from reference samples.

Preferably, the absolute value of the z-score of a sample aneuploid for the chromosome or chromosomal region of interest is above 4, still preferably above 4.4.

Preferably, the absolute value of the z-score of a sample euploid for the chromosome or chromosomal region of interest is below 4.4, still preferably below 4.

Preferably, the absolute value of the z-score of each sample of the reference set of samples is below 4.4, still preferably below 4.

In another embodiment, the comparison is carried out using a probability-based calculation. In this embodiment, a set of reference samples is used to determine an interval of values (discrimination thresholds T) which, in terms of probability, only a definite proportion of samples should exceed, for example but without being limited to one in 100, or one in 1'000, or one in 10'000, or one in 100'000 or one in 1'000'000 or one in 1'000'000'000. Where the discrimination thresholds are external, i.e. are derived from external reference samples, the process can comprise the following steps.

- A reference set of samples is provided, containing validated samples affected by the biological condition, as well as validated samples not affected by the biological condition (normal samples). The sequence count (for example the count of UEMs) of said samples are represented on a graph.

- the normal samples of the reference set are used to determine an interval of values (discrimination thresholds T) which, in terms of probability, which, in terms of probability, only a definite proportion of samples should exceed, for example one in 100, or one in 1'000, or one in 10'000, or one in 100'000 or one in 1'000'000 or one in 1'000'000'000. This interval is shown on the graph.

- then, the total amount of [N1 + N2] in the test sample is also indicated on the corresponding reference graph which serves as the basis for the clinical evaluation.

The tested sample is considered as positive for the biological condition if the test score exceeds the interval of values shown on the graph.

For more consistency in the determination, the process can be repeated several times using a plurality of reference sets, for example at least four and preferably six reference sets each comprising preferably at least 50 and preferably at least 75 reference samples. In such case, a tested sample may be considered as positive if, for every reference set used in the process, the test score exceeds the interval of values (threshold) shown on the graph.

Assessing the reliability of step (a)

A second step (b) of the method according to the invention comprises the comparison of the relative amount N1:N2 in the biological sample, to at least one discrimination threshold R. The role of step (b) is to assess of the reliability of the comparison of the total amount of [N1 + N2] to a discrimination threshold T, which is part of step (a). More generally, this second step is useful to assess the reliability of the method for determining the presence of a biological condition as a whole.

Determination of the relative amount N1:N2

In one embodiment, step (b) comprises the determination of the relative amount N1:N2 in the biological sample. The relative amount N1:N2 is for instance determined through the analysis of a distinguishing feature between N1 and N2, such as a sequence, an epigenetic state, the size, and/or the origin of the nucleic acid species.

In one embodiment, the relative amount N1:N2 is determined by detecting the presence of one or more nucleic acid fragments belonging to the nucleic acid species N1 or N2, such as fragments specific to the nucleic acid species N1 or N2. For example, in the case where N1 are fetal nucleic acids and N2 are maternal nucleic acids, the determination can be carried out by the quantification of target sequences specific to the fetus. If the fetus is a male fetus, the target sequences can be selected from the Y chromosome, such as the SRY gene. Alternatively, the target sequences can be selected from a set of known or presumable sequence variants, such as different alleles or SNPs. For example, where the nucleic acid species N1 is fetal nucleic acids, and the nucleic acid species N2 is maternal nucleic acids, the fetal fraction can be measured from the quantitative analysis of a fetal specific allele p, and of the corresponding maternal allele q.

$$FF = \frac{2p}{p+q} \quad (I)$$

The same Formula is applicable to cancer, where p corresponds to tumor-specific alleles, and q corresponds to the corresponding allele in non-tumor cells. Sets of several SNPs/variant alleles can be used for improving the statistical reliability of the fetal fraction determination (Chu et al., 2010; Sparks et al., 2012). In one embodiment, the procedure includes quantitative PCR (qPCR) and/or digital PCR (Lun et al., 2008), and/or target gene sequencing (Dawson et al., 2013). In another embodiment, the procedure includes the sequencing of at least a portion of the nucleic acids present in the biological sample, and the quantification of the target sequences (specific SNPs/alleles).

In another embodiment, e.g. in cases where the detected condition is cancer and N1 corresponds to tumor nucleic acids, whilst N2 corresponds to non-tumor nucleic acids, the tumor fraction, i.e. the proportion of cell-free tumor nucleic acids in the biological sample is assessable through the targeted sequencing analysis of SNPs exhibiting loss of heterogosity (LOH) (Chan et al., 2013). For such an analysis, a set of SNPs is chosen which exhibit LOH, as demonstrated for example from a SNP microarray analysis. The alleles deleted in the tumors have lower concentrations in the sample than those that the alleles that were not deleted, and the different in their concentrations is related to the concentration of tumor-derived nucleic acids in the sample. Thus, the concentration of the tumor-derived nucleic acids (tumor fraction, TF) can be deduced with the following equation:

$$TF = \frac{N_{nondel} - N_{del}}{N_{nondel}} \quad (II)$$

where $N_{nondel}$ represents the number of sequenced reads carrying the nondeleted alleles in the tumor tissues, and $N_{del}$ represents the number of sequenced reads carrying the deleted alleles in the tumor tissues.

In still another embodiment, the relative amount N1:N2 is determined by detecting epigenetic differences between N1 and N2. N1 and N2 are differentially methylated, and the relative amount N1:N2 is determined by using methylation-sensitive detection methods (as disclosed, for example, in the International patent publication WO2012/149339). In one embodiment, methylation-sensitive restriction enzymes are used.

In another embodiment, the relative amount N1:N2 is determined by using the fact that one of the nucleic acid species N1 or N2, or a subspecies thereof, is in lack or excess with respect to a discrimination threshold. Where N1 are fetal nucleic acids and N2 are maternal nucleic acids, the excess or lack may originate from a chromosomal or subchromosomal copy number variation. The chromosomal or subchromosomal is for example a chromosomal aneuploidy, such as trisomy 13, 18 or 21, or a benign or pathological subchromosomal copy number variation, e.g. a deletion, duplication or translocation.

For example, the fetal fraction FF can be inferred from the number of sequence tags mapped to an aneuploid chromosome 13, 18, 21, or from chromosome X in the case of male fetuses, and the number of sequence tags mapped to all chromosomes (see Fan and Quake, 2010). Formula III can be used:

$$ff = 2 \times \left| \frac{\overline{N_i}}{\overline{\overline{N}}} - 1 \right| \quad (III)$$

wherein $N_i$ is indicative of the sequence tag density on the ith chromosome, wherein the ith chromosome is affected by a fetal chromosomal aneuploidy, and is for example chromosome 13, 18 or 21, or wherein the ith chromosome is chromosome X in the case of a male sample; and

$N$ is indicative of the average global sequence tag density.

wherein the sequence tag density is a parameter indicative of the average sequence tag count in a fixed-size bin on a chromosome.

In an alternate embodiment, the fetal fraction in a sample affected by a chromosomal copy number variation, or in a male sample, is calculated by the Formula IV (as disclosed in Rava et al., 2014):

$$FF = 2 \times \left| \frac{R_{Ai}}{\overline{R_{Ui}}} - 1 \right| \quad (IV)$$

where $R_{Ai}$ is the normalized sequence tag count for an aneuploid chromosome i (for example chromosome 13, 18 or 21), or for chromosome X for a male sample; and $\overline{R_{Ui}}$ is the average normalized sequence tag count for the same chromosome i, calculated from n reference unaffected samples.

Whilst Formula III and IV are applicable only to samples from male fetuses, or to samples affected by a fetal chromosomal aneuploidy (i.e. monosomy or trisomy), i.e. approximately half of the samples normally analyzed in a routine NIPT test, another determination method has a broader applicability to any sample comprising a benign or pathological fetal subchromosomal copy number variation (Srinivasan et al., 2013). In this method, bins of fixed length (for example 100kb or 1 Mb) are defined, and the bin ratio value is calculated for each bin, according to Formula (V):

$$BRV_{ij} = \frac{Tags_{ij}}{\sum Tags_{km}} \quad (V)$$

Where $BRV_{ij}$ is the bin ratio value for the jth bin of chromosome i and $Tags_{ij}$ is the number of tags in the jth bin of chromosome i. The sum runs over a fixed number of bins with the nearest GC percentage to bin ij, for example 10 bins for 1Mb bins or 40 bins for 100 kb bins.

The $BRV_{ij}$ are then examined for deviations from the median values measured across multiple reference samples. The median absolute deviations (MADs) are calculated for each bin, based on the medians, and are adjusted assuming a normal distribution for the number of tags in each bin (i.e. MAD are multiplied by 1.4826), and are utilized to calculate a z score $z_{ij}$ for each bin (Formula VI):

$$z_{ij} = \frac{(BRV_{ij} - BRV_{Median_{ij}})}{aMAD_{ij}} \quad (VI)$$

For a bin ratio containing a copy-number change from number, the $BRV_{ij}$ will increase (in the case of a duplication) or decrease (in the case of a deletion), according to Formula VII:

$$BRV_{ij} = (1 \pm \frac{FF}{2}) BRV_{Median_{ij}} \quad (VII)$$

Where FF is the fetal fraction for the sample.

If we define the coefficient of variation for each bin, $CV_{ij}$ as

$$CV_{ij} = \frac{aMAD_{ij}}{BRV_{Median\,ij}} \quad (VIII)$$

then

$$FF = abs(2z_{ij}CV_{ij}) \quad (IX)$$

Formula IX can be used to calculate the fetal fraction from $z_{ij}$ values when a copy number variation is present. In comparison to the formulas above which necessitate the presence of a chromosomal aneuploidy, this formula extends the applicability of fetal fraction determination to a considerably greater amount of samples, i.e. approximately every sample.

The relative amount N1:N2 can be determined several times, using the same method for each determination, or using different methods. In such case, the mean value obtained from these several determinations, or any other statistically representative parameter, can be used for the purpose of comparing the relative amount N1:N2 to one or more discrimination thresholds R.

Discrimination threshold R

A further aspect of the method is the comparison of the determined relative amount N1:N2 with at least one discrimination threshold R, which is preferably specific for the method of step (a) according to the invention, in particular for the step of comparing the total amount [N1 + N2] to a discrimination threshold T.

The discrimination threshold is preferably determined empirically. In particular, it is obtained from one or more reference samples. It is preferably derived from the relative amount N1:N2 of the reference samples.

In one embodiment, the discrimination threshold R is indicative of the relative amount N1:N2 of a single reference sample. In order to derive the discrimination threshold R, the relative amount N1:N2 of the reference sample may be determined once. However, in order to increase the robustness of the discrimination threshold R, the relative amount is preferably determined several times on a same reference sample. For example, at least 10, 20, 50, 100, 200, 500 or 1000 determinations of the relative amount N1:N2 are carried out to define the discrimination threshold R. The several determinations are carried out by a same method or by different methods. Where there have been several determinations, the discrimination threshold R can be a parameter indicative of the determined relative amounts N1:N2, for example the mean of the relative amounts, or the mean $\pm$ one standard deviation, or two standard deviations, or three standard deviations, or any other statistically representative parameter.

The discrimination threshold R can also be defined using more than one reference sample. It is for instance indicative of the relative amounts N1:N2 of more than one reference sample, for example the mean, the mean $\pm$ one standard deviation, or two standard deviations, or three standard deviations, of the relative amounts N1:N2 of more than one reference sample. For each sample, the relative amount N1:N2 can be determined once. However, as described above, several determinations are preferred to increase the robustness of the discrimination threshold R.

Using reference samples as a source for the discrimination thresholds R means that in step (b), the test sample is compared to reference samples, with regard to the parameter "relative amount N1:N2". The method according to this embodiment is thus based on the use of discrimination threshold R obtained experimentally, which are specific for the method for determining the presence of a biological condition. This aspect of the method contrasts with the prior art in the field of NIPT, where a fetal fraction threshold, 4%, determined once and for all in a theoretical way, was used as a threshold for assessing whether a diagnosis method was reliable, irrespective of the diagnosis method actually implemented.

Selection of the reference samples for the relative amount N1:N2

The reference samples for the purpose of defining a discrimination threshold R are selected from a set of samples, on the basis of one or more selection criteria. Providing a high number of samples among which to select the reference samples will increase the statistical significance of the discrimination threshold. In one embodiment, the set of samples from which the reference samples are selected comprises at least 100 samples, preferably at least 200, 500, 1000, 2000, 5000, 10000, 20000, 50000 or 100000 samples.

The set of samples from which the reference samples are selected can also consist of samples which are 'positive' according to step (a), i.e. with a total amount [N1 + N2] above a discrimination threshold (T). Hence, in an embodiment, the set of samples from which the reference samples are selected comprises at least 10 samples with a total amount [N1 + N2] above a discrimination threshold T, preferably at least 20, 50, 100, 200, 500, 1000, 2000, 5000, 10000, 20000, 50000, 100000, 200000, 500000, or 1 million samples with a total amount [N1 + N2] above a discrimination threshold T.

The set of samples from which the reference samples are selected can also consist of samples affected by the biological condition. Hence, in an embodiment, the set of samples from which the reference samples are selected comprises at least 10 samples affected by the biological condition, preferably at least 20, 50, 100, 200, 500, 1000, 2000, 5000, 10000, 20000, 50000, 100000, 200000, 500000, or 1 million samples affected by the biological condition.

The selection of a reference sample can be performed on the basis of various criteria, including the following:

- whether the sample is affected by the biological condition/ is not affected by the biological condition.

- the total amount [N1 + N2] in the sample; for example, whether the total amount [N1 + N2] is above/below a discrimination threshold T (e.g. positive/negative with regard to the comparison carried out in step (a)); and/or the distance (e.g. the proximity) of the total amount [N1 + N2] to a discrimination threshold T;

- whether the diagnosis for a biological condition has been confirmed/validated by a different technique. Typically, in the case where the method for determining the presence of a biological condition is a NIPT method, such as MPS-based methods, the confirmation can be made by other non-invasive NIPT methods, such as ultrasound echography. The confirmation of the diagnosis can also be made by invasive techniques, such as amniocentesis or chorionic villus sampling, The confirmation of the initial diagnosis can also be made after the birth. In such a case, and if the biological condition is symptomatic, the confirmation may consist of a mere medical examination of the newborn child. In the case where the biological condition is cancer, the confirmation can be made by any method for diagnosing a cancer, including non-invasive diagnosis methods such as PET-scan, MRI, CT-scan, ultrasound imaging, x-ray imaging (e.g. mammography), or invasive diagnosis methods, such as a biopsy followed by an analysis (e.g. histopathological) of the removed tissue.

- The quality of the samples, as assessed, for example, by the number of sequences obtained in a massively parallel sequencing, the number of UESs, the size of the nucleic acid fragments.

In one embodiment, the reference sample is a sample with a total amount [N1 + N2] above a discrimination threshold T.

Alternatively or in addition, the reference sample is selected on the basis of the proximity of its total amount [N1 + N2] to a discrimination threshold T.

For example, the reference sample is selected from the samples, the value [N1+N2] of which is the most proximal to the discrimination threshold T, such as from the 0.01%, 0.1%,1%, 2%, 5%, 10%, 20% samples the value [N1+N2] of which is the most proximal to the discrimination threshold T, among a given set of samples. In another embodiment, the reference samples are selected from samples which diverge by at most 0.01%, 0.1 %,1 %, 2%, 5%, 10%, 20% from the discrimination threshold T.

In one embodiment, the reference sample is the sample with the lowest total amount [N1+N2], among all samples affected by the biological condition and having a total amount [N1 + N2] above the discrimination threshold T. This reference sample is considered as the sample with the lowest relative amount N1:N2, for which the biological condition can be reliably detected and defines a particular discrimination threshold R, referred to as a reliability threshold R, below which the comparison of method (a) is not considered reliable, and above which the comparison of method (a) is considered reliable.

In the field of NIPT, the reliability threshold R (which can be a fetal fraction threshold) of trisomy 13 can serve as reliability threshold R for other types of aneuploidies, such as trisomy 18 or 21. Indeed, the present inventors have confirmed that the fetal fraction in trisomy 13 pregnancies was in average lower than in trisomy 18 and 21 pregnancies, and have shown that this fetal fraction difference was reflected in the aneuploidy signal intensity of the samples (Figure 5). Hence, a trisomy 13 sample which defines a reliability threshold for trisomy 13 defines, at the same time, a reliability threshold for trisomy 18 and 21, and also for monosomy X (monosomy X pregnancies having also a overall higher fetal fraction than trisomy 13 pregnancies).

Once a reference sample has been selected, the robustness of the selection can be verified. For example, where the total amount [N1 + N2] is used as a criterion for selecting a reference sample, several determinations of this total amount can be performed to verify, for instance, that for each determination, [N1 + N2] is determined above the discrimination threshold T, i.e. that every time, the sample is detected as positive for the biological condition. In the same way, it can be verified that for each determination, the sample fulfils the defined selections criterion (criteria). For example, several dominations of [N1 + N2] are carried out to verify that for each determination the selected reference sample has a value [N1 + N2] which is among the most proximal to a discrimination threshold T, for example the sample belongs to the 0.01%, 0.1%,1%, 2%, 5%, 10%, 20% samples the value [N1+N2] of which is the most proximal to a discrimination threshold T.

Where the method for determining the presence of a biological condition is a MPS-based method, the robustness of the selection can be verified by preparing a new sequencing library, sequencing the nucleic acid species N1 and N2, mapping the sequences to a reference sequence, and determining the total amount [N1 + N2] to compare it with the discrimination threshold T.

Alternatively, the same sequencing library as that used initially can be resequenced, followed by a mapping of the sequences, and a determination of the total amount [N1 + N2] to compare it with the discrimination threshold T.

Several reference samples can be selected, either to derive a single discrimination threshold R, for example from the relative amounts N1:N2 of several reference samples, or to derive several discrimination thresholds R, e.g. one from every reference sample. In this way, a full scale of discrimination thresholds R, originating from a set of reference samples, can be used as a basis for comparison of the relative amount N1:N2 of the test sample.

Comparison of the relative amount N1:N2 of the test sample with the discrimination threshold R

The comparison of the relative amount N1:N2 of the test sample with a discrimination threshold R can involve assessing whether the relative amount N1:N2 of the test sample is above or under the discrimination threshold R. The comparison can also involve assessing the distance between the relative amount N1:N2 of the tested sample and the discrimination threshold R. Where there are several discrimination thresholds R, the comparison can involve situating the relative amount N1:N2 on a scale of discrimination threshold R.

The main purpose of the comparison carried out in step (b) is to validate the reliability of the comparison carried out in step (a), in such a way to validate the determination of the biological condition.

The determination of the biological condition is for example validated if the relative amount N1:N2 of the test sample is above one or several discrimination thresholds R, such as a reliability threshold R and/or is positioned sufficiently high in a scale of discrimination thresholds R.

In such situations, the relative amount N1:N2 is hypothesized to have no causal implication on the outcome of step (a) and/or, more generally, on the outcome of the method for determining the presence of a biological condition. In such a case, step (a), and/or, more generally the method for determining the presence of a biological condition is assumed to be reliable, at least with regards to the parameter "relative amount N1:N2".

Accordingly, a positive outcome of step (b), i.e. the comparison of N1:N2 with the discrimination threshold R allows the validation of step (a), i.e. the comparison of [N1+N2] with the discrimination threshold T.

Conversely, the determination of the biological condition may not be validated if the relative amount N1:N2 of the test sample is below one or several discrimination thresholds R, such as a reliability threshold R, and/or is positioned sufficiently low in the scale of discrimination thresholds R. In such a case, it can be hypothesized that the relative amount N1:N2 in the test sample is insufficient, and this insufficiency is suspected to have a causal implication on the outcome of the method for determining the presence of a biological condition. Hence, the reliability of the method for determining the presence of a biological condition is questioned, at least with regards to the parameter "relative amount N1:N2".

Alternatively or in addition, the discrimination threshold R is used to assess the strength/intensity of the relative amount N1:N2. The assessment may be quantitative and/or qualitative. The sample is for example rated as having a very low, low, moderate, high, or very high relative amount N1:N2 on the basis of a comparison with one or more discrimination threshold(s) R.

In one embodiment, further valuable information can be drawn from the two comparisons carried out for [N1 + N2] and N1:N2, especially where there exists a discrepancy between the outcome of both comparisons. For instance, valuable information is provided by analyzing the correlation, or lack of correlation between the total amount [N1+ N2] and the relative amount N1:N2. The inventors have indeed noticed the existence, in typical cases of chromosomal aneuploidies (trisomy 13, 18 or 21, for example) of a correlation between the total amount [N1+N2] (i.e. the "aneuploidy" signal intensity, or in other terms, the count of sequences mapped to a chromosome) and the fetal fraction (i.e. the relative amount N1:N2). Hence, the presence or lack of such a correlation is informative with regard to the biological condition. Accordingly, an embodiment of the method according to the invention includes analyzing the correlation between the total amount [N1+N2] and the relative amount N1:N2 and deducing from said analysis information on the biological condition and/or on the patient. In an embodiment, analyzing the correlation between the total amount [N1+N2] and the relative amount N1:N2 includes comparing this correlation with reference values for the correlation and/or, wherein these reference values are obtained, for instance, from reference samples affected or not by the biological condition. In another embodiment, analyzing the correlation between the total amount [N1+N2] and the relative amount N1:N2 includes comparing the total amount [N1 + N2] with one or more reference values, such as one or more discrimination thresholds T, and comparing the relative amount N1:N2 with one or more reference values, such as one or more discrimination thresholds R. In the field of NIPT, examples of information or conditions which can be revealed by the analysis of the correlation between [N1+N2] and N1:N2 include, without being limited to the following cases:

- A high fetal fraction (N1:N2), e.g. 25%, does not correlate with a too low signal strength for aneuploidy ([N1+N2]). This can reveal a twin pregnancy with one of the two fetuses being affected from an aneuploidy, or a pre-eclampsia.

- a high signal strength for aneuploidy ([N1+N2]) does not correlate with a too low fetal fraction (N1:N2), e.g. a fetal fraction below 3%, 5% or 7%. This can reveal a mother herself affected from an aneuploidy.

- The fetal fraction (N1:N2) does not correlate with a too low signal strength for aneuploidy ([N1+N2]). This can reveal a fetus affected from a true fetal mosaicism.

In an embodiment, where the relative amount N1:N2 is found insufficient, measures can be taken to improve the reliability of the method for determining the presence of a biological condition. The same measures can be implemented even though the relative amount N1:N2 is found sufficient in a first test, e.g. to verify the outcome of this first test.

One of these measures can be the redrawing of a sample from the patient, followed by a further implementation of the whole method for determining the presence of the biological condition, on the new sample. Sometimes, the insufficient amount N1:N2 in the initial sample is related to an insufficient quality of the initial sample, e.g. due to a problem at the drawing stage, and/or during the storing of the sample, and/or during the nucleic acid extraction. The drawing of a new sample will normally permit to overcome the problem and obtain a meaningful diagnosis. Where the patient is a pregnant woman, and the nucleic acid species N1 consists of cell-free nucleic acids from the fetus, it may be useful to redraw the new sample at a later stage in the course of pregnancy. Indeed, the concentration of cell-free fetal DNA (fetal fraction) in the maternal blood is demonstrated to increase over the course of pregnancy, and by waiting a certain period of time before the redrawing of a blood sample, one can expect to have a higher fetal fraction in the new sample. The time period between the initial drawing and the redrawing is for example of at least one week, or at least two weeks, or at least one month, or at least two months, or at least three months. Similarly, in the case of cancer detection, waiting for a certain period of time before a further drawing can enrich the amount of tumor-derived nucleic acids, e.g. cell-free tumor-derived nucleic acids in the biological sample, in particular in the blood sample of the patient. The time period between the initial drawing and the redrawing is for example of at least one week, or at least two weeks, or at least one month, or at least three months, or at least six months.

The detection of the presence of the biological condition can also be improved by enriching the relative amount N1:N2 in the initial biological sample, for example by enriching the nucleic acid species N1, and/or by depleting the nucleic acid species N2.

In one embodiment, nucleic acid species N1 is enriched by selective amplification, for example by PCR. The amplification can be selective for a sequence, as well as for an epigenetic state, such as methylation, as described in the International patent publication WO2010/033639.

Alternatively, nucleic acid species N1 is enriched by affinity selection, for example on a column, or on a microarray. Affinity selection may include a step of hybridization.

In another embodiment, the enrichment is carried out by size selection.

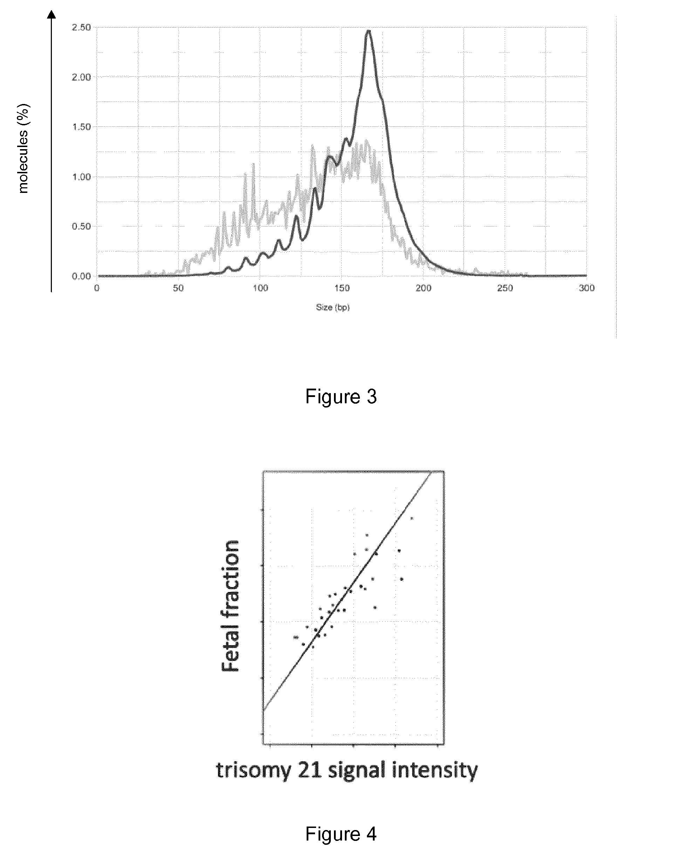

Size selection has been shown to be an effective way to enrich the fetal fraction of a maternal sample containing both cell-free fetal DNA and cell-free maternal DNA. Fetal fraction enrichment by size selection is based on the findings that the size distribution of cell-free fetal DNA differs from the size distribution of maternal cell free DNA (Figure 3). Size distribution of maternal DNA shows a peak at -166 bp in length, whilst this peak is downshifted for fetal nucleic acids, i.e. the 166 bp peak is reduced, and a 143 bp peak is increased. Without being limited by theory, this difference, as well as the particular size distribution of cell free DNA is thought to be related to its origin: the 166 bp peak is assumed to correspond to the size of a nucleosome core particle of -146 bp plus a linker histone of 20 bp. The 143 bp peak would correspond to the nucleosome core particle trimmed from the ~20bp linker histone. This size distribution could reflect that plasma DNA fragments are derived from the enzymatic processing of DNA from apoptotic cells (Lo et al., 2010).

Size selection is also an efficient way to enrich cell-free tumor nucleic acids (in particular DNA), i.e. increase the relative amount of cell-free tumor nucleic acids over cell-free non tumor nucleic acids. Indeed, tumor cell-free DNA fragments are reported to be smaller in size than non-tumor cell-free DNA fragments (Mouliere et al., 2014). A selection of small fragments, e.g. of less than 100 nucleotides thus allows the enrichment of tumor cell-free DNA.

In one embodiment, nucleic acid species N1 is enriched, in the sample, by enriching, e.g. by size selection, the nucleic acid molecules with a maximum length of less than 300 nucleotides, or less than 200 nucleotides, or less than 150 nucleotides, or less than 100 nucleotides. Molecules of between 40 and 200 nucleotides, or between 50 and 150 nucleotides can also be enriched, e.g. by size selection.

Technically, size selection can be carried out by electrophoresis, in particular by capillary electrophoresis or gel electrophoresis. In another embodiment, size selection is performed by affinity capture, e.g. by purification on beads. The use of magnetic beads is particularly preferred, for example AMPure XP® beads. According to this technology, DNA fragments bind to the magnetic beads, and are then separated from contaminants by application of a magnetic field. The bound DNA is washed with ethanol and is then eluted from the magnetic particles.

Enrichment of N1 can be performed at any time before the sequencing. In one embodiment, the enrichment is carried out concurrently with the extraction of the nucleic acids, for instance when the nucleic acids are extracted by purification on beads. Alternatively, or in addition, the enrichment is performed after the nucleic acid extraction, and before the preparation of the sequencing library, or during the preparation of the sequencing library, or after the preparation of the sequencing library and before the sequencing.

Examples

**Example 1 : Workflow for aneuploidy determination**

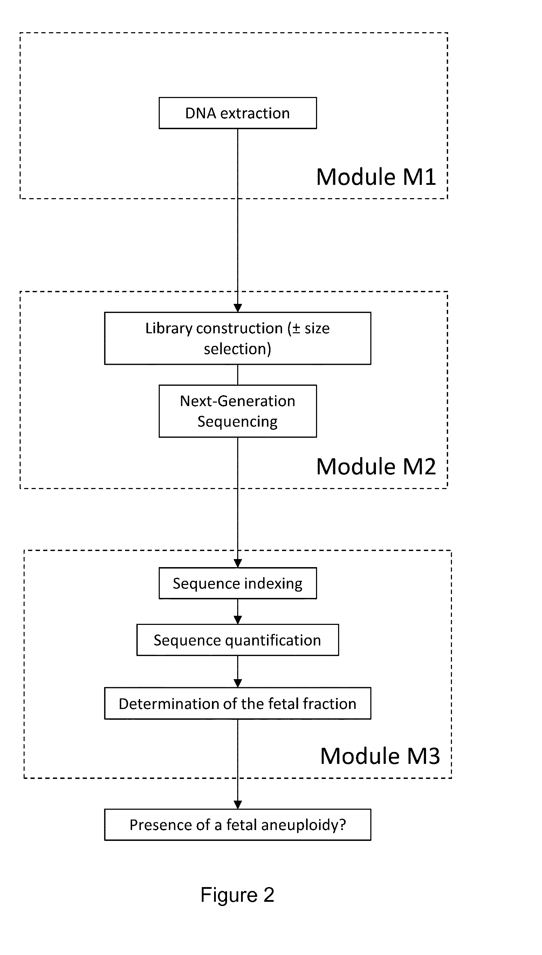

The workflow is based on three technical modules M1 to M3 (Figure 2).

In the first module M1, plasma is separated from whole blood through a double spin, and total cell-free DNA is extracted, either with the help of a manual method using phenolchloroform extraction and alcohol-based precipitation, or alternatively using an automated method comprising magnetic beads. Total DNA is then subjected to the process of module M2 comprising the library preparation that can be manual of fully automatized. During this process, adaptors that contain primer sites for amplification, sequencing and indexing are being ligated to cell-free DNA. A step of size selection - in order to enrich the fetal fraction and/or to eliminate large fragments of the size class >150nt - can be performed before or after the amplification step during library preparation. The size selection can involve capillary electrophoresis of amplified library products and size selection on agarose gels, with methods such as excision of labeled DNA during electrophoresis based on size comparison with size standards. Alternatively, during other steps of library construction, large DNA fragments of the size-class of >150 nucleotides are eliminated with a procedure involving purification on beads. The optionally size-selected library products are massively parallel sequenced using one of the commercial platforms such as Illumina sequencers or Ion proton sequencers with a shotgun procedure. The third module M3 is the bioinformatics analysis that consists of two main processes: the first process attributes the sequenced cell-free DNA molecules of the size class of 50 nucleotides and larger to individual chromosomes (indexing), which then allows quantification. Discrimination of a normal karyotype from aneuploidy is achieved with the help of reference sets (see Figure 5, 7 or 8A, for example). Samples from pregnancies with normal karyotype constitute the "normal reference group" and samples from pregnancies with an aneuploidy karyotype (e.g. trisomy 13, 18, or 21), constitute the "aneuploid reference group".

These "unaffected" and "affected" reference groups allow the definition of discrimination thresholds T (dotted lines) used to discriminate for a given clinical sample whether it contains two copies of the chromosome in question (bold spot on Figure 7A)) or whether it is affected by aneuploidy, for example showing three copies of the chromosome in question (trisomy 13, 18 or 21) or one chromosome (monosomy X). The thresholds T of '$10^{-3}$' or '$10^{-4}$' are defined by using the reference samples, such that a random sample will have a statistical probability of 1/1'000, respectively 1/10'000 of exceeding the threshold.

The former analysis is carried out for every autosome and chromosome X, using 6 different reference sets for each chromosome. This process allows the drawing of a genome-wide view of the lack or excess of sequences, in the sample (Figure 9C, for example). Each lane represents a chromosome, and in every lane, each bar represents a comparison with a reference set. A chromosome which exceeds the threshold of $10^{-3}$ for all 6 reference sets is considered as aneuploid (either trisomic or monosomic depending on the presence of a lack or excess of sequences for the relevant chromosome). The second process of module M3 is the determination of the fetal fraction and the comparison to a fetal fraction threshold, as described in examples 3 to 6 below.

The outcomes of the two bioinformatics processes are then used to issue a diagnosis, i.e. whether the fetus is affected by an aneuploidy at one of the analyzed chromosomes.

**Example 2 : Correlation between the fetal fraction and the signal intensity for aneuploidy**

A set of reference samples affected from trisomy 21 was used to analyze the correlation between fetal fraction and the signal intensity for trisomy 21 (the signal intensity reflects the total count of maternal and fetal cell-free DNA sequences mapping to chromosome 21, as described in Example 1). Figure 4 shows that there is a clear correlation between fetal fraction and signal intensity for trisomy 21. Similar findings are made for other types of aneuploidy (trisomies 18 and 13, monosomy X).

Recent reports show that the fetal fraction varies among aneuploidies (Dar et al.). Trisomy 21 pregnancies have a fetal fraction slightly higher than euploid pregnancies (+5%), whereas trisomy 18 (-10%), monosomy X (-15%) and trisomy 13 pregnancies (-25%) have a fetal fraction lower than euploid pregnancies. By using reference samples affected by trisomies 13, 18 and 21, the inventors assessed whether the differences of fetal fraction among aneuploidies were reflected in the signal intensity, i.e. the intensity of 'aneuploidy calling' signal, derived from the count of sequences mapped to a chromosome. As illustrated in Figure 5, the samples with the lowest signal intensity (among positive samples, i.e. samples which exceed the aneuploidy threshold) are closer to the $10^{-3}$ or $10^{-4}$ threshold in trisomy 13 pregnancies, than in trisomy 18 and 21 pregnancies. In other terms, the fetal fraction differences among aneuploidies are reflected in the signal intensity of the samples. Hence, by selecting trisomy 13 samples having the lowest signal intensity to set a reliable fetal fraction threshold, you i) ensure that your threshold is adapted to the biologically lowest fetal fraction, and ii) automatically ensure that you will have higher power of detecting the other major aneuploidies.

**Example 3 : Selection of a fetal fraction threshold**

Sample GWX-311 was detected as positive for trisomy 13 and had, among the samples positive for a fetal chromosomal aneuploidy, the lowest signal. In other terms, sample GWX-311 was the closest from the $10^{-3}$ threshold used to define a samples as positive for a chromosomal aneuploidy.

47 independent analyses were performed to confirm the classification of GWX-311 as positive for trisomy 13. The mean score of these 47 independent analyses was compared to 6 independent reference sets of samples. As shown in Figure 6, chromosome 13 was the only chromosome to be called in a concordant way by all 6 independent reference sets, thus confirming the robustness of the diagnosis of trisomy 13 on sample GWX-311.

Sample GWX-311 was thus selected as a reference sample for defining a discrimination threshold R serving as a fetal fraction threshold for the detection of fetal aneuploidy.

Overall 110 replicated analyses of sample GWX-311 were used to determine the fetal fraction of the sample. The mean - 2 SD (mean minus 2 standard deviations) of the 110 individual predictions of the fetal fraction was selected as the fetal fraction threshold R, corresponding to a fetal fraction of 3%.

The robustness of this fetal fraction threshold was assessed with the help of seven samples with a confirmed valid NIPT result, and a known fetal fraction value (as determined by counting sequences with a known fetal origin) distributed across the scale of values. TPR-2709 was specifically selected to represent the upper boundary of high fetal fractions. At the other extreme of the scale, TPR-2429 was selected to represent a sample with a false negative trisomy 18 testing and bona fide too low fetal fraction. FDT-41, FDT-44, FDT-40, FDT-42, FDT43 and FDT-39 all represent samples with a correct NIPT result.

TPR-2709, FDT-41, FDT-44, FDT-40, FDT-42, FDT43 and FDT-39 are all correctly determined to be above the fetal fraction threshold R, i.e. the fetal fraction in these samples does not adversely affect the reliability of the NIPT result, which correlates with the fact that the NIPT result for these samples is confirmed valid. On the other hand, TRP-2429 is correctly determined to be below the fetal faction threshold R, i.e. the fetal fraction in this sample is insufficient to guarantee a reliable NIPT result.

This example shows the robustness of the fetal fraction threshold selected by the empirical method according to the invention. Clinical decision making can then be implemented for samples that are below the threshold. For instance, one can suggest a second blood draw that will naturally enrich the fetal fraction because the gestational age increased, or, more technically, enrich the fetal fraction experimentally (see Example 4)

**Example 4 : work-up of false negative results encountered during the diagnostic procedure**

Two NIPT results were reported back as being false negative, one concerning sample number GWX-2429 (Figure 7A), the other the sample number GWX-2488 (Figure 8A). In both cases, the nominal result produced was "no evidence for aneuploidy, female fetus". However, fetal anomalies were found on ultrasound examination in the further post-test course of the pregnancy. In both cases an amniocentesis was performed and karyotyping revealed for the two of them the presence of a standard trisomy 18 (47, XX, +18).

In order to be able to implement preventive measures, the present inventors tried to elucidate the reason for those false negative results. The strategy to attain this aim was the following:

(i) search for known biological sources of false negative results
(ii) formulation of a hypothesis concerning the potential cause(s)
(iii) design of experiments to disprove the hypothesis
(iv) conclusion
v) implementation of measures.

(i) For sample GWX-2488 a known risk factor for a low fetal fraction was readily identified, namely a maternal weight of 110 kg. The ethnic origin was south European/Greek. This implies according to the literature that 3-5% of pregnancies from women with such a weight will have a fetal fraction below the threshold considered critical, i.e. 4%; (Canick et al., 2013; Wang et al., 2013). For the other sample, GWX-2429, of Caucasian origin no known risk factor could be identified.

(ii) The hypotheses formulated were then the following:

(a) a low fetal fraction was responsible for the false negative result;
(b) alternative factors are involved.

(iii) The experimental design was as follows. The fetal fraction for both samples was determined, and compared to the threshold R defined as in Example 1, corresponding to the fetal fraction of known trisomy 13 sample (GWX-311), with a low fetal fraction, but still compatible with a reproducibly robust detection of trisomy 13, and thereby of trisomies 18 and 18, as trisomy 13 is known to be associated with a lower fetal fraction than the other major trisomies. GWX-2429 but not GWX-2488 was predicted to have a fetal fraction below the lower threshold defined by GWX-311. GWX-2488 in turn was predicted to have a fetal fraction in the lower normal range (percentiles P7-P15), a range that typically is compatible with a highly robust detection of aneuploidy.

The fetal fraction for both samples was enriched to a similar extent, and both samples were tested (Figures 7B and 8B/C, respectively) to assess whether trisomy 18 now became detectable according to the defined standard diagnostic criteria (sample above the $10^{-3}$ threshold in 6 comparisons against 6 different reference sample sets). Enrichment of the fetal fraction of sample GWX-2429 restored the canonical detection of trisomy 18, proving the causal implication of the fetal fraction in the false negative result. By way of contrast, similar enrichment of GWX-2488 did not restore detection of trisomy 18, although the significance level of warnings (i.e. the number of comparisons exceeding $10^{-3}$) had increased overall (from $<1 \times 10^{-3}$ to $2 \times 10^3$).

iv) The false negative result for GWX-2429 is hypothesized to be due to an extremely low fetal fraction of unknown origin without clear risk factors. The false negative result for GWX-2488 is not due to a low fetal fraction. According to the literature and compatible with the inventors' data, the most likely explanation is a true fetal mosaicism (TFM). In the latter case, the aneuploidy cell line is not present in the cytotrophoblast at all, or at least not in a sufficient amount to allow reliable detection even at the highest fetal fractions.

v) As measures to be taken, one is to implement the measurement of the fetal fraction in the context of a functional concept how to handle samples with fetal fractions lower than the reliably detected lower threshold. As regard TFM, the literature anticipates that false negative results due to a TFM will occur in the order of 1/3'000 (Grati et al., 2014). This possible cause of false negative results should be explored when fetal fraction is not suspected to be liable for the false negative result.

## Example 5 : Diagnosis including a determination of the fetal fraction

The diagnosis method according to the invention was assessed on two samples DPA-3932 (Figure 9) and GWX-3522 (Figure 10), obtained from two different pregnant women.

DPA-3932 is negative for all chromosomal aneuploidies (Figure 9C), in particular for trisomy 13 (Figure 9B). Moreover, the fetal fraction of this sample, as estimated via 10 determinations, exceeds the lower experimental threshold indicated by the lower dotted line (Figure 9A).

DPA-3932 is thus diagnosed negative for all chromosomal aneuploidies. The estimated fetal fraction validates the diagnosis.

GWX-3522 is positive for trisomy 13 (6 comparisons exceeding the $10^{-3}$ threshold, Figure 10C), as also shown in Figure 10B, showing a single comparison with a reference set, for chromosome 13. Moreover, the fetal fraction of this sample, as estimated via 10 determinations, exceeds the lower experimental threshold indicated by the lower dotted line (Figure 10A).

GWX-3522 is thus diagnosed positive for trisomy 13. The estimated fetal fraction validates the diagnosis.

## Example 6 : Correlation or discrepancy between the signal intensity and **the** fetal fraction

The correlation of the fetal fraction with the signal intensity for aneuploidy was assessed on two samples TPV-113 (Figure 11) and TPV-116 (Figure 12), from two different pregnant women. For both samples, the fetus is affected by trisomy 21, as detected by a result of 6 comparisons exceeding the $10^{-3}$ threshold for both samples (Figures 11A and 12A).

Sample TPV-113 has a very strong signal intensity, exceeding the 1/1'000'000'000 threshold for all 6 comparisons with 6 different reference sets of samples (Figure 11A). This strong signal correlates with a high fetal fraction, as shown in Figure 11B, showing the result from 10 determinations of the fetal fraction.

Sample TPV-116, although correctly diagnosed for trisomy 21, has a lower signal intensity, which exceeds the 1/1'000'000'000 threshold for only 2 reference sets (Figure 12A). This lower signal correlates with a lower fetal fraction, as shown in Figure 12B, showing the result from 10 determinations of the fetal fraction.

These two samples illustrate the natural correlation between fetal fraction and the signal intensity for the sequence count. Failure to meet this correlation, i.e. a discrepancy between the fetal fraction and the signal intensity for the sequence count can be indicative of an "anomaly", such as a mother affected from trisomy, a fetus affected from a true fetal mosaicism, or a twin pregnancy.

As an example of such discrepancy, sample GWX-2488, discussed in Example 4, has a fetal fraction in the normal range, whilst the signal intensity for trisomy 18 is too low to allow a diagnosis of the disease. Furthermore, enrichment of the fetal fraction does not restore a canonical detection of trisomy 18, although the levels of warnings are increased. This discrepancy is indicative of a true fetal mosaicism.

Another discrepancy sample, sample DPA-4670, had a signal intensity exceeding the 1/1'000 threshold for the 6 reference sets (Figure 13A). The signal intensity was however too low in comparison with a fetal fraction of 11% (Figure 13B). A twin pregnancy and a vanishing twin affected by trisomy 21 was suspected. Effectively, the demise of an affected co-twin and the survival of the non-affected co-twin were substantiated.

## Example 7 : Enrichment of the fetal fraction

Sample DPA-4006 from a pregnant woman was diagnosed positive for the Turner syndrome, i.e. monosomy X. The signal strength for chromosome X was however low, and close to the diagnosis threshold (Figure 14A). Similarly, fetal fraction was above the minimal threshold, which would validate the diagnosis, but it was at the same time low (Figure 14B).

In order to verify the accuracy of this diagnosis, a new blood sample was drawn two weeks later. The fetal fraction was enriched (Figure 14D), as well as the signal strength (Figure 14C), allowing a clear and unquestioned diagnosis of monosomy X, for this sample.

Another sample, DPA-4215, was diagnosed negative for the Turner syndrome (Figure 15A). The fetal fraction was however below the minimal threshold (Figure 15 B). Hence, the diagnosis was not validated. In order to provide a reliable diagnosis, a new blood sample was drawn two weeks later. The fetal fraction was enriched (Figure 15D). At the same time, the aneuploidy signal was still between the thresholds defining an euploid sample, which classifies sample DPA-4215 as diploid for the chromosome X (Figure 15C).

Another way to enrich the fetal fraction is illustrated in Figure 16: size selection, which consists in the elimination of DNA molecules over a certain size, for example 150 nucleotides. Figure 16 shows the results of an automated DNA extraction, including an enrichment by size selection (protocol D), as compared to a manual protocol of DNA extraction (protocol A), and 3 automated protocols of DNA extraction which do not include a size selection procedure (protocols B, C and E). In this example, size selection allows a fetal fraction enrichment of 3-5 fold.

The foregoing specification, including the specific embodiments and examples, is intended to be illustrative of the present invention and is not to be taken as limiting. Numerous other variations and modifications can be effected without departing from the true spirit and scope of the present invention.

## Bibliography

Ashoor, G., Poon, L., Syngelaki, A., Mosimann, B., and Nicolaides, K.H. (2012). Fetal fraction in maternal plasma cell-free DNA at 11-13 weeks' gestation: effect of maternal and fetal factors. Fetal Diagn. Ther. 31, 237-243.

Ashoor, G., Syngelaki, A., Poon, L.C.Y., Rezende, J.C., and Nicolaides, K.H. (2013). Fetal fraction in maternal plasma cell-free DNA at 11-13 weeks' gestation: relation to maternal and fetal characteristics. Ultrasound Obstet. Gynecol. Off. J. Int. Soc. Ultrasound Obstet. Gynecol. 41, 26-32.

Beroukhim, R., Mermel, C.H., Porter, D., Wei, G., Raychaudhuri, S., Donovan, J., Barretina, J., Boehm, J.S., Dobson, J., Urashima, M., Mc Henry, K.T., Pinchback, R.M., Ligon, A.H., Cho, Y.-J., Haery, L., Greulich, H., Reich, M., Winckler, W., Lawrence, M.S., Weir, B.A., 2010. The landscape of somatic copy-number alteration across human cancers. Nature 463, 899-905. doi:10.1038/nature08822

Bianchi, D.W. (2012). From prenatal genomic diagnosis to fetal personalized medicine: progress and challenges. Nat. Med. 18, 1041-1051.

Cai, X., Evrony, G.D., Lehmann, H.S., Elhosary, P.C., Mehta, B.K., Poduri, A., Walsh, C.A., 2014. Single-Cell, Genome-wide Sequencing Identifies Clonal Somatic Copy-Number Variation in the Human Brain. Cell Reports 8, 1280-1289. doi:10.1016/j.celrep.2014.07.043

Canick, J.A., Palomaki, G.E., Kloza, E.M., Lambert-Messerlian, G.M., and Haddow, J.E. (2013). The impact of maternal plasma DNA fetal fraction on next generation sequencing tests for common fetal aneuploidies. Prenat. Diagn. 33, 667-674.

Chan, K.C.A., Jiang, P., Zheng, Y.W.L., Liao, G.J.W., Sun, H., Wong, J., Siu, S.S.N., Chan, W.C., Chan, S.L., Chan, A.T.C., et al. (2013). Cancer genome scanning in plasma: detection of tumor-associated copy number aberrations, single-nucleotide variants, and tumoral heterogeneity by massively parallel sequencing. Clin. Chem. 59, 211-224.

Chu, T., Bunce, K., Hogge, W.A., and Peters, D.G. (2010). A novel approach toward the challenge of accurately quantifying fetal DNA in maternal plasma. Prenat. Diagn. 30, 1226-1229.

Dar, P., Curnow, K.J., Gross, S.J., Hall, M.P., Stosic, M., Demko, Z., Zimmermann, B., Hill, M., Sigurjonsson, S., Ryan, A., et al. Clinical experience and follow-up with large scale single-nucleotide polymorphism-based noninvasive prenatal aneuploidy testing. Am. J. Obstet. Gynecol.

Dawson, S.-J., Tsui, D.W.Y., Murtaza, M., Biggs, H., Rueda, O.M., Chin, S.-F., Dunning, M.J., Gale, D., Forshew, T., Mahler-Araujo, B., et al. (2013). Analysis of Circulating Tumor DNA to Monitor Metastatic Breast Cancer. N. Engl. J. Med. 368, 1199-1209.

Fan, H.C., and Quake, S.R. (2010). Sensitivity of noninvasive prenatal detection of fetal aneuploidy from maternal plasma using shotgun sequencing is limited only by counting statistics. PloS One 5, e10439.

Frazer, K.A., Murray, S.S., Schork, N.J., and Topol, E.J. (2009). Human genetic variation and its contribution to complex traits. Nat. Rev. Genet. 10, 241-251.

Grati, F.R., Malvestiti, F., Ferreira, J.C.P.B., Bajaj, K., Gaetani, E., Agrati, C., Grimi, B., Dulcetti, F., Ruggeri, A.M., De Toffol, S., et al. (2014). Fetoplacental mosaicism: potential implications for false-positive and false-negative noninvasive prenatal screening results. Genet. Med. Off. J. Am. Coll. Med. Genet. 16, 620-624.

Hui, L., and Bianchi, D.W. (2013). Recent advances in the prenatal interrogation of the human fetal genome. Trends Genet. TIG 29, 84-91.

Lo, Y.M., Corbetta, N., Chamberlain, P.F., Rai, V., Sargent, I.L., Redman, C.W., and Wainscoat, J.S. (1997). Presence of fetal DNA in maternal plasma and serum. Lancet 350, 485-487.

Lun, F.M.F., Tsui, N.B.Y., Chan, K.C.A., Leung, T.Y., Lau, T.K., Charoenkwan, P., Chow, K.C.K., Lo, W.Y.W., Wanapirak, C., Sanguansermsri, T., et al. (2008). Noninvasive prenatal diagnosis of monogenic diseases by digital size selection and relative mutation dosage on DNA in maternal plasma. Proc. Natl. Acad. Sci. U. S. A. 105, 19920-19925. Mouliere, F., El Messaoudi, S., Pang, D., Dritschilo, A., Thierry, A.R., 2014. Multi-marker analysis of circulating cellfree DNA toward personalized medicine for colorectal cancer. Mol Oncol 8, 927-941. doi:10.1016/j.molonc.2014.02.005

Rava, R.P., Srinivasan, A., Sehnert, A.J., and Bianchi, D.W. (2014). Circulating fetal cell-free DNA fractions differ in autosomal aneuploidies and monosomy X. Clin. Chem. 60, 243-250.

Sparks, A.B., Wang, E.T., Struble, C.A., Barrett, W., Stokowski, R., McBride, C., Zahn, J., Lee, K., Shen, N., Doshi, J., et al. (2012). Selective analysis of cellfree DNA in maternal blood for evaluation of fetal trisomy. Prenat. Diagn. 32, 3-9.

Srinivasan, A., Bianchi, D.W., Huang, H., Sehnert, A.J., and Rava, R.P. (2013). Noninvasive detection of fetal subchromosome abnormalities via deep sequencing of maternal plasma. Am. J. Hum. Genet. 92, 167-176.

Struble, C.A., Syngelaki, A., Oliphant, A., Song, K., and Nicolaides, K.H. (2014). Fetal fraction estimate in twin pregnancies using directed cell-free DNA analysis. Fetal Diagn. Ther. 35, 199-203.

Wang, E., Batey, A., Struble, C., Musci, T., Song, K., and Oliphant, A. (2013). Gestational age and maternal weight effects on fetal cell-free DNA in maternal plasma. Prenat. Diagn. 33, 662-666.

## Claims

1. Method for determining the presence of a biological condition associated with a nucleic acid species N1 in a test biological sample obtained from a patient, said test biological sample comprising two nucleic acid species N1 and N2, wherein the method comprises:

   a) determining the total amount [N1 + N2] and comparing said total amount to at least one discrimination threshold T;
   b) determining the relative amount N1:N2 and comparing said relative amount to at least one discrimination threshold R specific for the method of step (a); and
   c) on the basis of the comparisons carried out in step (a) and (b), determining the presence of the biological condition.

2. Method according to claim 1, wherein the comparison in step (b) involves comparing the relative amount N1:N2 of the test biological sample to the relative amount N1:N2 of one or more reference samples.

3. Method according to claim 2, wherein the reference samples are selected by comparing their total amount [N1 + N2] with respect to the discrimination threshold T.

4. Method according to any one of claims 1 to 3, wherein the nucleic acid species N1 and N2 are cell free nucleic acids.

5. Method according to any one of claims 1 to 4, wherein the nucleic acid species N1 are fetal nucleic acids and the nucleic acid species N2 are maternal nucleic acids, or wherein the nucleic acids N1 are derived from a tumor cell and/or tissue, and the nucleic acid species N2 are derived from a non-tumor cell and/or tissue.

6. Method according to any one of claims 1 to 5, wherein, if the relative amount N1:N2 is below the discrimination threshold R:

- the relative amount N1:N2 is enriched; and/or
- the biological condition is detected on another biological sample drawn from the same patient.

7. Method according to claim 6, wherein the relative amount N1:N2 is enriched by size selection of the nucleic acids in the test biological sample, preferably by enriching the nucleic acids with a maximum length of 200 nucleotides or less, or 175 nucleotides or less, or 150 nucleotides or less.

8. Method according to any one of claims 1 to 7, wherein determining the total amount [N1 + N2] comprises:

    - sequencing the nucleic acids species N1 and N2 from the test biological sample;
    - aligning the sequences to a reference genome or a portion thereof; and
    - from the aligned sequences, determining the total amount [N1 + N2].

9. Method according to any one of claims 1 to 8, wherein the comparison in step (a) involves comparing the total amount [N1 + N2] of the test biological sample to the total amount [N1 + N2] of one or more reference samples.

10. Method according to any one of claims 1 to 9, wherein the biological condition is cancer or a fetal chromosomal or subchromosomal copy number variation, preferably trisomy 13, 18 or 21.

11. Method according to claim 10, wherein the biological condition is a fetal chromosomal copy number variation, and the comparison in step (b) involves comparing the relative amount N1:N2 of the test biological sample to the relative amount N1:N2 of one or more reference samples obtained from a trisomy 13 pregnancy.

12. Method for defining a discrimination threshold R for a relative amount N1:N2 of two nucleic acid species N1 and N2 in a test biological sample, said discrimination threshold R being specific for a method for determining the presence of a biological condition associated with N1, said method for determining the presence of a biological condition comprising the comparison of the total amount [N1 + N2] in the test biological sample to at least one discrimination threshold T, wherein said method for defining a discrimination threshold R comprises providing at least one reference sample, determining the relative amount N1:N2 in the reference sample, and defining the discrimination threshold R from the relative amount N1:N2 in the reference sample.

13. Method according to claim 12, wherein the reference sample is selected by comparing its total amount [N1 + N2] with the discrimination threshold T.

14. Method according to claim 12 or 13, wherein the biological condition is a fetal chromosomal copy number variation and the reference sample is obtained from a trisomy 13 pregnancy.

15. Method for assessing the reliability of a method for determining the presence of a biological condition associated with a nucleic acid species N1 in a test biological sample comprising two nucleic acid species N1 and N2, said method for determining the presence of a biological condition comprising the comparison of the total amount of [N1 + N2] to at least one discrimination threshold T, wherein the reliability of the method for determining the presence of a biological condition is assessed by comparing the relative amount N1:N2 in the test biological sample to at least one discrimination threshold R specific for the method for determining the presence of a biological condition.

16. Method for determining the presence of a biological condition associated with a nucleic acid species N1 in a test biological sample obtained from a patient, comprising two nucleic acid species N1 and N2, said method comprising:

    - providing a set of biological samples obtained from a set of patients;
    - selecting at least one reference sample from the set of biological samples by comparing its total amount [N1 + N2] with at least one discrimination threshold T;
    - determining the total amount [N1+N2] in the test biological sample and comparing said total amount to the discrimination threshold T;
    - determining the relative amount N1:N2 in the test biological sample and comparing said relative amount to the relative amount N1:N2 in the reference sample(s); and
    - on the basis of the results of both comparisons, determining the presence of the biological condition.

Figure 1

```
┌──────────────────────────────────────────────────────┐
│                                                        │
│            ┌─────────────────────┐                     │
│            │   DNA extraction    │                     │
│            └─────────────────────┘                     │
│                                            Module M1   │
└──────────────────────────────────────────────────────┘

┌──────────────────────────────────────────────────────┐
│         ┌─────────────────────────────┐               │
│         │ Library construction (± size│               │
│         │         selection)          │               │
│         └─────────────────────────────┘               │
│         ┌─────────────────────────────┐               │
│         │      Next-Generation        │               │
│         │        Sequencing           │               │
│         └─────────────────────────────┘               │
│                                            Module M2   │
└──────────────────────────────────────────────────────┘

┌──────────────────────────────────────────────────────┐
│           ┌─────────────────────────┐                 │
│           │    Sequence indexing    │                 │
│           └─────────────────────────┘                 │
│         ┌────────────────────────────┐                │
│         │   Sequence quantification  │                │
│         └────────────────────────────┘                │
│      ┌──────────────────────────────────┐             │
│      │ Determination of the fetal fraction│           │
│      └──────────────────────────────────┘             │
│                                            Module M3   │
└──────────────────────────────────────────────────────┘

        ┌──────────────────────────────────┐
        │  Presence of a fetal aneuploidy? │
        └──────────────────────────────────┘
```

Figure 2

Figure 3

Figure 4

A.        B.        C.

Figure 5

Figure 6

A.

B.

Figure 7

A.

B.

C.

Figure 8

A.

B.

C.

Figure 9

A.

B.

C.

Figure 10

A.

B.

Figure 11

A.

B.

Figure 12

A.

B.

Figure 13

Figure 14

C.

D.

Figure 14 (continued)

A.

B.

Figure 15

C.

D.

Figure 15 (continued)

Figure 16

**EUROPEAN SEARCH REPORT**

Application Number

EP 14 19 1764

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KYPROS H. NICOLAIDES ET AL: "Assessment of Fetal Sex Chromosome Aneuploidy Using Directed Cell-Free DNA Analysis", FETAL DIAGNOSIS AND THERAPY, vol. 35, no. 1, 1 January 2014 (2014-01-01), pages 1-6, XP055183965, ISSN: 1015-3837, DOI: 10.1159/000357198 * page 2, right-hand column * * page 2, left-hand column, last paragraph * | 1,3-5,8 | INV. C12Q1/68 |
| X | FAN H-M C: "MOLECULAR COUNTING:FROM NONINVASIVE PRENATAL DIAGNOSTICS TOWHOLE-GENOME HAPLOTYPING", INTERNET CITATION, 1 November 2010 (2010-11-01), pages I-XVII, XP002675933, Retrieved from the Internet: URL:https://stacks.stanford.edu/file/druid:cw095xw9265/thesisfinal-augmented.pdf [retrieved on 2012-05-08] * pages 61,81; figure 3.9 * | 1,4-10, 12,13, 15,16 | |

-----

-/--

**TECHNICAL FIELDS SEARCHED (IPC)**

C12Q

The present search report has been drawn up for all claims

| Place of search | Date of completion of the search | Examiner |
|---|---|---|
| Munich | 20 April 2015 | Knudsen, Henrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 14 19 1764

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TAK Y. LEUNG ET AL: "Noninvasive twin zygosity assessment and aneuploidy detection by maternal plasma DNA sequencing", PRENATAL DIAGNOSIS, vol. 33, no. 7, 29 April 2013 (2013-04-29), pages 675-681, XP055125576, ISSN: 0197-3851, DOI: 10.1002/pd.4132 * page 677, left-hand column, paragraphs 1,4 * * page 676, left-hand column * * page 678, right-hand column, lines 9-11 * * page 677, right-hand column, paragraph 2 * | 1,4,5, 8-10 | |
| X | JACOB A. CANICK ET AL: "The impact of maternal plasma DNA fetal fraction on next generation sequencing tests for common fetal aneuploidies", PRENATAL DIAGNOSIS, vol. 33, no. 7, 31 July 2013 (2013-07-31), pages 667-674, XP055127544, ISSN: 0197-3851, DOI: 10.1002/pd.4126 * page 670; figure 2 * | 1,2,4,5 | TECHNICAL FIELDS SEARCHED (IPC) |
| X | R. P. RAVA ET AL: "Circulating Fetal Cell-Free DNA Fractions Differ in Autosomal Aneuploidies and Monosomy X", CLINICAL CHEMISTRY, vol. 60, no. 1, 17 September 2013 (2013-09-17), pages 243-250, XP055125837, ISSN: 0009-9147, DOI: 10.1373/clinchem.2013.207951 * page 7, right-hand column, last paragraph; table 1 * | 1,11,12, 14 | |

-/--

The present search report has been drawn up for all claims

| Place of search | Date of completion of the search | Examiner |
|---|---|---|
| Munich | 20 April 2015 | Knudsen, Henrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 14 19 1764

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/014497 A1 (VERINATA HEALTH INC [US]; RAVA RICHARD P [US]; CHINNAPPA MANJULA [US];) 23 January 2014 (2014-01-23) * claim 1; example 60 * | 1 | |
| A | GARY J. W. LIAO ET AL: "Noninvasive Prenatal Diagnosis of Fetal Trisomy 21 by Allelic Ratio Analysis Using Targeted Massively Parallel Sequencing of Maternal Plasma DNA", PLOS ONE, vol. 7, no. 5, 29 May 2012 (2012-05-29), page e38154, XP055062111, DOI: 10.1371/journal.pone.0038154 * page 2, right-hand column, paragraph 2 * | 1-16 | |
| A | WO 2014/015319 A1 (VERINATA HEALTH INC [US]) 23 January 2014 (2014-01-23) * page 162, paragraph 1 * | 1-16 | |
| A | WO 2014/068075 A1 (GENESUPPORT SA [CH]) 8 May 2014 (2014-05-08) * pages 46-47 * | 1-16 | TECHNICAL FIELDS SEARCHED (IPC) |
| T | WO 2014/205401 A1 (SEQUENOM INC [US]) 24 December 2014 (2014-12-24) * claims 1,41,47 * | | |

The present search report has been drawn up for all claims

| Place of search | Date of completion of the search | Examiner |
|---|---|---|
| Munich | 20 April 2015 | Knudsen, Henrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 14 19 1764

This annex lists the patent family members relating to the patent documents cited in the above-mentioned European search report.
The members are as contained in the European Patent Office EDP file on
The European Patent Office is in no way liable for these particulars which are merely given for the purpose of information.

20-04-2015

| Patent document cited in search report | | Publication date | Patent family member(s) | | Publication date |
|---|---|---|---|---|---|
| WO 2014014497 | A1 | 23-01-2014 | AU | 2013204615 A1 | 06-02-2014 |
| | | | CA | 2878246 A1 | 23-01-2014 |
| | | | WO | 2014014497 A1 | 23-01-2014 |
| | | | WO | 2014014498 A1 | 23-01-2014 |
| WO 2014015319 | A1 | 23-01-2014 | US | 2014038830 A1 | 06-02-2014 |
| | | | WO | 2014015319 A1 | 23-01-2014 |
| WO 2014068075 | A1 | 08-05-2014 | EP | 2728014 A1 | 07-05-2014 |
| | | | WO | 2014068075 A1 | 08-05-2014 |
| WO 2014205401 | A1 | 24-12-2014 | US | 2015005176 A1 | 01-01-2015 |
| | | | WO | 2014205401 A1 | 24-12-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014068075 A
- WO 2012149339 A
- WO 2010033639 A

### Non-patent literature cited in the description

- **ASHOOR, G. ; POON, L. ; SYNGELAKI, A. ; MOSI-MANN, B. ; NICOLAIDES, K.H.** Fetal fraction in maternal plasma cell-free DNA at 11-13 weeks' gestation: effect of maternal and fetal factors. *Fetal Diagn. Ther.,* 2012, vol. 31, 237-243
- **ASHOOR, G. ; SYNGELAKI, A. ; POON, L.C.Y. ; REZENDE, J.C. ; NICOLAIDES, K.H.** Fetal fraction in maternal plasma cell-free DNA at 11-13 weeks' gestation: relation to maternal and fetal characteristics. *Ultrasound Obstet. Gynecol. Off. J. Int. Soc. Ultrasound Obstet. Gynecol.,* 2013, vol. 41, 26-32
- **BEROUKHIM, R. ; MERMEL, C.H. ; PORTER, D. ; WEI, G. ; RAYCHAUDHURI, S. ; DONOVAN, J. ; BARRETINA, J. ; BOEHM, J.S. ; DOBSON, J. ; URASHIMA, M.** The landscape of somatic copy-number alteration across human cancers. *Nature,* 2010, vol. 463, 899-905
- **BIANCHI, D.W.** From prenatal genomic diagnosis to fetal personalized medicine: progress and challenges. *Nat. Med.,* 2012, vol. 18, 1041-1051
- **CAI, X. ; EVRONY, G.D. ; LEHMANN, H.S. ; ELHO-SARY, P.C. ; MEHTA, B.K. ; PODURI, A. ; WALSH, C.A.** Single-Cell, Genome-wide Sequencing Identifies Clonal Somatic Copy-Number Variation in the Human Brain. *Cell Reports,* 2014, vol. 8, 1280-1289
- **CANICK, J.A. ; PALOMAKI, G.E. ; KLOZA, E.M. ; LAMBERT-MESSERLIAN, G.M. ; HADDOW, J.E.** The impact of maternal plasma DNA fetal fraction on next generation sequencing tests for common fetal aneuploidies. *Prenat. Diagn.,* 2013, vol. 33, 667-674
- **CHAN, K.C.A. ; JIANG, P. ; ZHENG, Y.W.L. ; LIAO, G.J.W. ; SUN, H. ; WONG, J. ; SIU, S.S.N. ; CHAN, W.C. ; CHAN, S.L. ; CHAN, A.T.C. et al.** Cancer genome scanning in plasma: detection of tumor-associated copy number aberrations, single-nucleotide variants, and tumoral heterogeneity by massively parallel sequencing. *Clin. Chem.,* 2013, vol. 59, 211-224
- **CHU, T. ; BUNCE, K. ; HOGGE, W.A. ; PETERS, D.G.** A novel approach toward the challenge of accurately quantifying fetal DNA in maternal plasma. *Prenat. Diagn.,* 2010, vol. 30, 1226-1229
- **DAR, P. ; CURNOW, K.J. ; GROSS, S.J. ; HALL, M.P. ; STOSIC, M. ; DEMKO, Z. ; ZIMMERMANN, B. ; HILL, M. ; SIGURJONSSON, S. ; RYAN, A. et al.** Clinical experience and follow-up with large scale single-nucleotide polymorphism-based noninvasive prenatal aneuploidy testing. *Am. J. Obstet. Gynecol.*
- **DAWSON, S.-J. ; TSUI, D.W.Y. ; MURTAZA, M. ; BIGGS, H. ; RUEDA, O.M. ; CHIN, S.-F. ; DUNNING, M.J. ; GALE, D. ; FORSHEW, T. ; MAHLER-ARAUJO, B. et al.** Analysis of Circulating Tumor DNA to Monitor Metastatic Breast Cancer. *N. Engl. J. Med.,* 2013, vol. 368, 1199-1209
- **FAN, H.C. ; QUAKE, S.R.** Sensitivity of noninvasive prenatal detection of fetal aneuploidy from maternal plasma using shotgun sequencing is limited only by counting statistics. *PloS One,* 2010, vol. 5, e10439
- **FRAZER, K.A. ; MURRAY, S.S. ; SCHORK, N.J. ; TOPOL, E.J.** Human genetic variation and its contribution to complex traits. *Nat. Rev. Genet.,* 2009, vol. 10, 241-251
- **GRATI, F.R. ; MALVESTITI, F. ; FERREIRA, J.C.P.B. ; BAJAJ, K. ; GAETANI, E. ; AGRATI, C. ; GRIMI, B. ; DULCETTI, F. ; RUGGERI, A.M. ; DE TOFFOL, S. et al.** Fetoplacental mosaicism: potential implications for false-positive and false-negative noninvasive prenatal screening results. *Genet. Med. Off. J. Am. Coll. Med. Genet.,* 2014, vol. 16, 620-624
- **HUI, L. ; BIANCHI, D.W.** Recent advances in the prenatal interrogation of the human fetal genome. *Trends Genet. TIG,* 2013, vol. 29, 84-91
- **LO, Y.M. ; CORBETTA, N. ; CHAMBERLAIN, P.F. ; RAI, V. ; SARGENT, I.L. ; REDMAN, C.W. ; WAINSCOAT, J.S.** Presence of fetal DNA in maternal plasma and serum. *Lancet,* 1997, vol. 350, 485-487

- **LUN, F.M.F. ; TSUI, N.B.Y. ; CHAN, K.C.A. ; LEUNG, T.Y. ; LAU, T.K. ; CHAROENKWAN, P. ; CHOW, K.C.K. ; LO, W.Y.W. ; WANAPIRAK, C. ; SANGUANSERMSRI, T. et al.** Noninvasive prenatal diagnosis of monogenic diseases by digital size selection and relative mutation dosage on DNA in maternal plasma. *Proc. Natl. Acad. Sci. U. S. A.,* 2008, vol. 105, 19920-19925
- **MOULIERE, F. ; EI MESSAOUDI, S. ; PANG, D. ; DRITSCHILO, A. ; THIERRY, A.R.** Multi-marker analysis of circulating cell-free DNA toward personalized medicine for colorectal cancer. *Mol Oncol,* 2014, vol. 8, 927-941
- **RAVA, R.P. ; SRINIVASAN, A. ; SEHNERT, A.J. ; BIANCHI, D.W.** Circulating fetal cell-free DNA fractions differ in autosomal aneuploidies and monosomy X. *Clin. Chem.,* 2014, vol. 60, 243-250
- **SPARKS, A.B. ; WANG, E.T. ; STRUBLE, C.A. ; BARRETT, W. ; STOKOWSKI, R. ; MCBRIDE, C. ; ZAHN, J. ; LEE, K. ; SHEN, N. ; DOSHI, J. et al.** Selective analysis of cell-free DNA in maternal blood for evaluation of fetal trisomy. *Prenat. Diagn.,* 2012, vol. 32, 3-9
- **SRINIVASAN, A. ; BIANCHI, D.W. ; HUANG, H. ; SEHNERT, A.J. ; RAVA, R.P.** Noninvasive detection of fetal subchromosome abnormalities via deep sequencing of maternal plasma. *Am. J. Hum. Genet.,* 2013, vol. 92, 167-176
- **STRUBLE, C.A. ; SYNGELAKI, A. ; OLIPHANT, A. ; SONG, K. ; NICOLAIDES, K.H.** Fetal fraction estimate in twin pregnancies using directed cell-free DNA analysis. *Fetal Diagn. Ther.,* 2014, vol. 35, 199-203
- **WANG, E. ; BATEY, A. ; STRUBLE, C. ; MUSCI, T. ; SONG, K. ; OLIPHANT, A.** Gestational age and maternal weight effects on fetal cell-free DNA in maternal plasma. *Prenat. Diagn.,* 2013, vol. 33, 662-666